# EUROPEAN PATENT APPLICATION

(11) **EP 4 692 348 A1**
(43) Date of publication of application: **11.02.2026**
(21) Application number: 24382889.4
(22) Date of filing: 07.08.2024
(51) Int. Cl.: C12N 15/113

(54) **ARTIFICIAL RNAS FOR TREATING REPEAT EXPANSION DISORDERS**

(71) Applicant: Nocturna Therapeutics SL, 08018 Barcelona (ES); Universidad Pompeu Fabra, 08002 Barcelona (ES)
(72) Inventor: DOTU RODRIGUEZ, Ivan Javier, 08018 Barcelona (ES); TALLÓ PARRA, Marc, 08018 Barcelona (ES); DÍEZ ANTON, Juana, 08002 Barcelona (ES)
(74) Representative: Hoffmann Eitle

(57) **Abstract**

The present invention relates to an artificial RNA molecule capable of hybridizing to a target mRNA, wherein the target mRNA is characterized by comprising (a) pathological nucleotide repeat expansions, and (b) at least one secondary structure, wherein the artificial RNA molecule is capable of disrupting by hybridization the secondary structure. Kits, compositions and uses thereof are also provided.

## Description

### FIELD OF THE INVENTION

The present invention relates to the field of biomedicine. In particular, the present invention relates to artificial RNAs suitable for disrupting by hybridization one or more secondary structures of one or more target mRNAs, thereby treating repeat expansion disorders.

### BACKGROUND OF THE INVENTION

Repeat expansion disorders are a class of genetic diseases that are caused by expansions in DNA repeats. The DNA repeats come in various sizes from single nucleotides to dodecamers or longer. The degree of within-generation and across-generation change varies among the disorders, in part because the size of expansions varies from less than 30 repeats in some disease to upwards of one or several thousand repeats in other diseases. Expanded trinucleotide repeat diseases were discovered first and remain the most frequent. More recently tetra-, penta-, hexa- and even dodeca-nucleotide repeat expansions have been identified as the cause of human disease, including some of the most common genetic disorders seen by neurologists. Repeat expansion diseases include both causes of myotonic dystrophy (DM1 and DM2), the most common genetic cause of amyotrophic lateral sclerosis/frontotemporal dementia (C9ORF72), Huntington disease and eight other polyglutamine disorders including the most common forms of dominantly inherited ataxia, the most common recessive ataxia (Friedreich ataxia), and the most common heritable mental retardation (Fragile X Syndrome).

The threshold at which the repeat expansions become symptomatic varies with the specific disease. There are over 40 distinct diseases now known to be caused by these expansions in DNA sequence, and remarkable progress during the last three decades has defined causative mutations that drive pathogenesis in a large number of these diseases.

Repeat expansions are inherently dynamic, often changing size when transmitted to the next generation. As a result, even in the same family, the phenotypic range of disease in affected individuals can vary remarkably.

The diseases tend to show a striking genotype-phenotype correlation between repeat length and disease severity. The longer the repeat, the more severe the disease and the earlier the onset of symptoms. The fact that these diseases are due to a single, specific expanded repeat simplifies genetic testing.

So far, the treatment of nucleotide repeat disorders has been mainly complication-oriented, with no actual treatment for the disorder itself. Some clinical trials are being conducted to find an effective way of blocking the translation of the aberrant protein. Other treatments are based on protein inhibitors, antagonists and stem cell therapy. However, the new genetic tools available open the path for new therapies capable of acting at a DNA or RNA level.

### BRIEF DESCRIPTION OF THE DRAWINGS

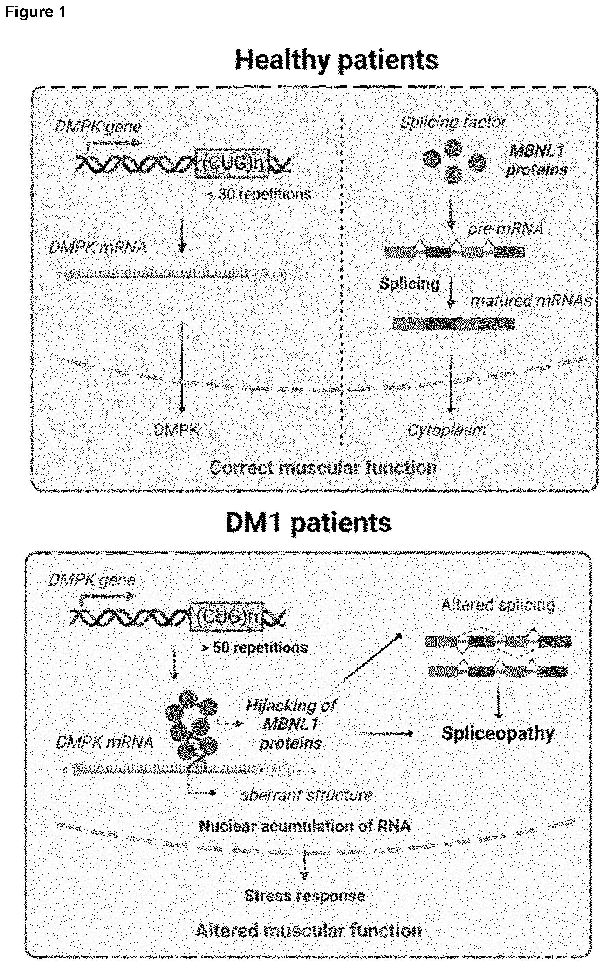

**Figure 1****:** Schematic model of the molecular basis of the pathogenesis of Steinert's disease. In healthy patients (left), the DMPK gene is transcribed into messenger RNA (mRNA) encoding the DMPK protein. In parallel, MBNL1 splicing factors promote the splicing of different precursors to mature mRNA that will go to the cytoplasm to be translated and promote proper muscle function. In patients with Steinert (right), the DMPK gene exhibits CTG trinucleotide expansion of 50 to 3000 repeats. When the DMPK gene is transcribed, an aberrant structure is formed in the RNA molecule that sequesters the key splicing factors (MBNL1) generating alternative and aberrant splicing processes in multiple mRNAs that will lead to spliceopathy. In addition, the formation of this aberrant structure induces the accumulation of these RNAs in the nucleus generating a stress response. The combination of splicing defects, the accumulation of RNAs in the nucleus and the activation of stress response signaling cascades will promote impaired muscle function and the onset of disease symptoms.

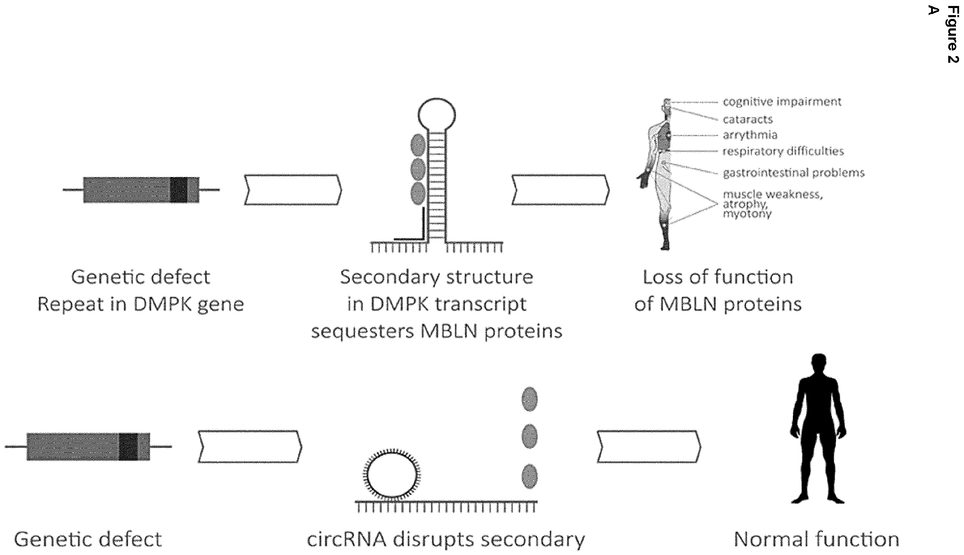

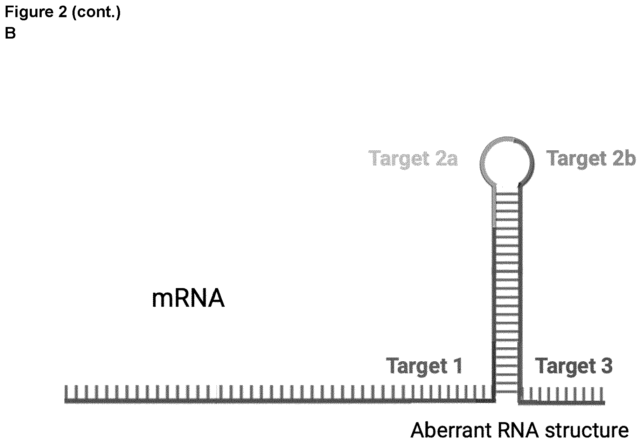

**Figure 2:** A) Mechanism of action of the circRNAs against Steinert disease. B) CircRNA targeted regions in DM 1 aberrant structures. CircRNA binding sites consist of a single stranded RNA followed by a double stranded RNA region, belonging to the targeted structure. The three possible target regions in DM1 aberrant structures are depicted in different shadings.

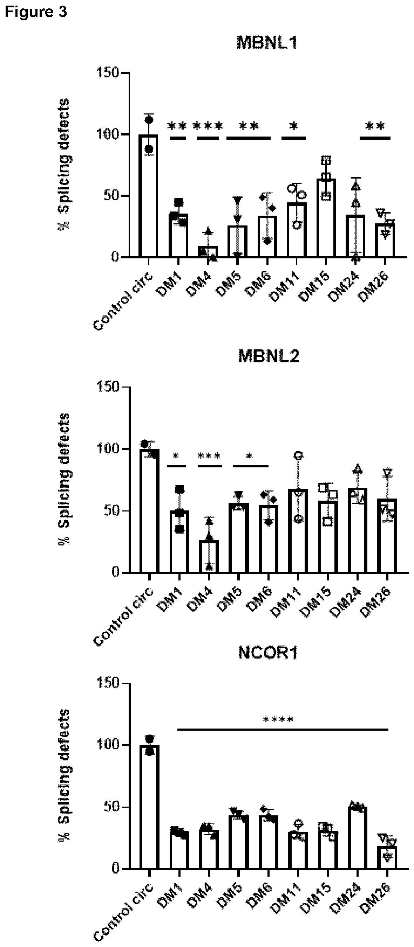

**Figure 3****:** DM1 splicing defects are restored in cells treated with circRNAs. Splicing defects of MBNL1, MBNL2 and NCOR2 transcripts, are represented as percentage of abnormal transcripts of three biological replicates (±SEM) related to a negative control (NC). Statistical significance was calculated using a one-way ANOVA test, (*represents p-value < 0.05).

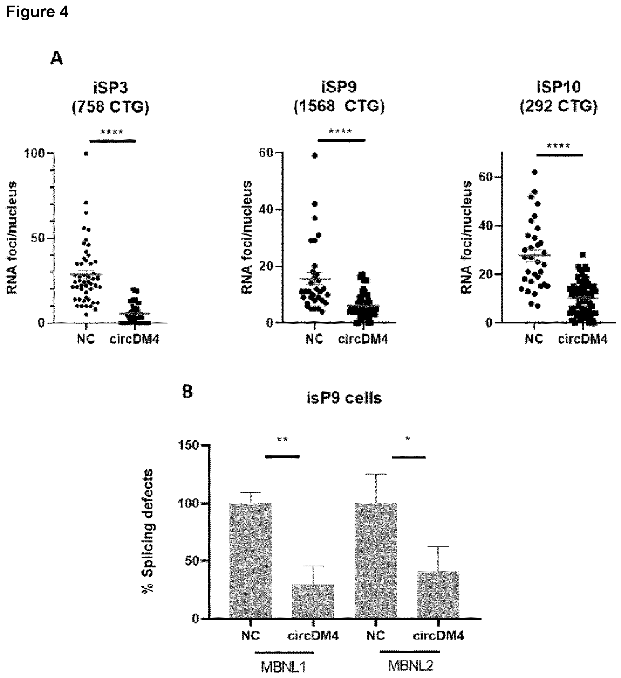

**Figure 4****: CircDM4 restores cellular alteration of DM1 patient derived myocytes.** A) Reduction of pathogenic nuclear focis in DM1 patient myocyte cell lines, treated with circDM4, Results are represented as total number of focis normalized by the total number of observed nucleus. NC corresponds to a negative control. Statistical significance was calculated using a T-test (*represents p-value < 0.05). B) MBNL1 and MBNL2 splicing defects are restored in DM1 patient derived myocytes treated with circDM4. Splicing defects are represented as percentage of MBNL1 or MBNL2 abnormal transcripts of three biological replicates (±SEM) related to a negative control (NC). Statistical significance was calculated using a T-test (*represents p-value < 0.05).

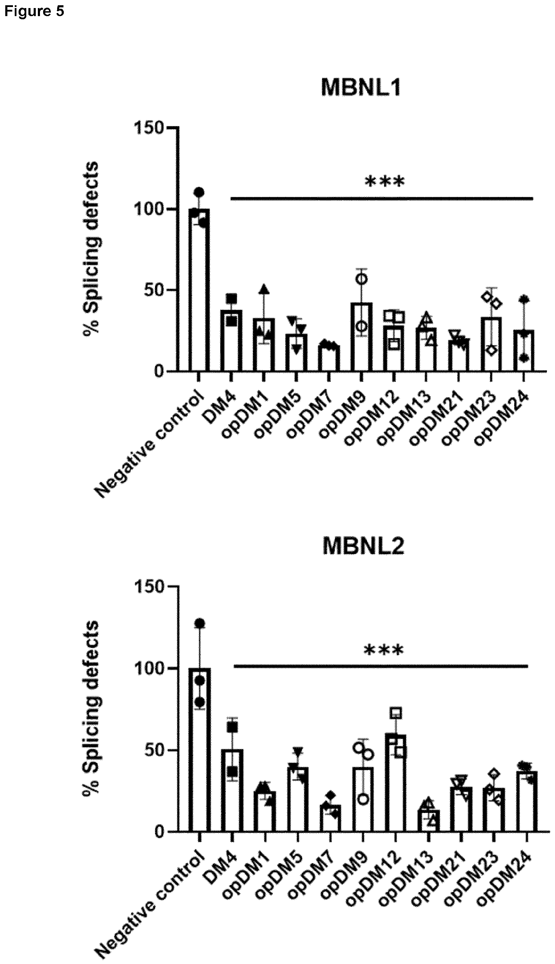

**Figure 5****: DM1 splicing defects are restored in cells treated with optimized circRNAs.** Splicing defects of MBNL1 and MBNL2 transcripts are represented as a percentage of abnormal transcripts of three biological replicates (±SEM) related to a negative control (NC).

### DETAILED DESCRIPTION OF THE INVENTION

### Definitions

In describing the present disclosure, the following terms will be used and are defined as indicated below.

The forms "a", "an" and "the" include plural referents unless the context states otherwise.

The term "about" when referred to a given amount or quantity is meant to include deviations of plus or minus five percent.

The term "artificial circular RNAs" or "circular RNAs" or "circRNAs" is used herein to refer to non-coding RNAs molecules forming covalently closed continuous loops which have the 3' and 5' ends joint together, thus they are missing the 5'-cap and in consequence the polyadenylated tail. Therefore, they are resistant to exonuclease-mediated degradation and to debranching enzymes, which confers them higher stability with respect to other RNAs.

The term "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% (or total) complementary refers to the situation in which each nucleotide unit of one polynucleotide strand or region can hydrogen bond with each nucleotide unit of a second polynucleotide strand or region. Less than perfect (or partial) complementarity refers to the situation in which some, but not all, nucleotide units of two strands or two regions can hydrogen bond with each other and can be expressed as a percentage.

The term "hybridization" is used to refer to the structure formed by 2 independent strands of RNA that form a double stranded structure via base pairings from one strand to the other. These base pairs are considered to be G-C, A-U and G-U. (A - Adenine, C -Cytosin, G-Guanine, U - Uracil). As in the case of the complementarity, the hybridization can be total or partial.

The term "transfection or "transfect" is used to refer to the uptake of RNA by a cell. A cell has been "transfected" when the RNA has been introduced inside the cell membrane.

"Stem-loop intramolecular base pairing" ("stem-loops") is a pattern that can occur in single-stranded DNA or, more commonly, in RNA. The structure is also known as a "hairpin" or "hairpin loop". It occurs when two regions of the same strand, usually complementary in nucleotide sequence when read in opposite directions, base-pair to form a double helix that ends in an unpaired loop. The resulting structure is a key building block of many RNA secondary structures. The base-pairing of the helix need not be complete, there could be stretches of single stranded nucleotides, which are called "bulges" or "internal loops". As an important secondary structure of RNA, it can direct RNA folding, protect structural stability for messenger RNA (mRNA), provide recognition sites for RNA binding proteins, and serve as a substrate for enzymatic reactions.

"Internal-loops" (also termed "interior loops"), in RNA, are found where the double stranded RNA separates due to no Watson-Crick base pairing between the nucleotides. Internal-loops can be classified as either symmetrical or asymmetrical, with some asymmetrical internal-loops, also known as bulges. Internal-loops differ from stem-loops as they occur in the middle of a stretch of double stranded RNA. "External-loops" are stretches of single-stranded nucleotides that separate hairpin loops or multi-loops. "Multi-loops" are branching off of a double-stranded region into several hairpin loops.

"Disruption by hybridization": given a secondary structure, a target hybridization region and an artificial RNA that completely hybridizes with the target hybridization region, disruption by hybridization occurs when the energy of the hybridization is more favourable than the energy of the secondary structure (more negative). Upon such hybridization, the structure of the secondary structure changes, at least, completely for the overlap between the secondary and the target hybridization region. Such energy calculations can be obtained in silico using well established software like RNAcofold, RNAstructure, Mfold or Vienna package, see below.

The term 5'UTR refers to the region upstream of the coding region, immediately before the start codon in a given RNA. The term 3'UTR refers to the region of a given RNA that is located downstream of the CDS, immediately after the stop codon. The term CDS refers to the coding region of a given mRNA.

"Administering" an artificial RNA to a cell comprises transducing, transfecting, electroporating, translocating, fusing, phagocytosing, shooting or ballistic methods, etc., i.e., any means by which a nucleic acid can be transported across a cell membrane.

The degree of identity between two sequences can be determined by conventional methods, for example, by means of standard sequence alignment algorithms known in the state of the art, such as, for example BLASTn (Altschul S.F. et al. Basic local alignment search tool. J Mol Biol. 1990 Oct 5; 215(3):403-10).

During the description of the claims, the word "*comprising*", and its variants does not intend to exclude other technical characteristics, additives, components or steps. In addition, the term "*comprising*" may also encompass the term "*consisting of*"*.*

### DESCRIPTION OF THE INVENTION

Unstable DNA repeat expansions have been found to cause more than 50 neurodevelopmental, neurodegenerative, and neuromuscular disorders. One of the main hallmarks of repeat expansion diseases is the formation of abnormal RNA or protein aggregates in the neuronal cells of affected individuals. A common feature of those diseases is the presence of pathological mRNA molecules that carry the nucleotide expansions and form secondary structures that alter their function. The present inventors have designed artificial RNAs, preferably circular RNAs, comprising at least one (and preferably more than one) hybridization region that hybridizes to a region within secondary structures and disrupt them, thereby inhibiting the pathological function of said target mRNA.

### The artificial RNA of the invention

In **a first aspect,** the present invention provides an artificial RNA molecule, also called herein "the artificial RNA of the invention" that is capable of hybridizing to a pathological target mRNA, wherein the target mRNA is characterized by having pathological nucleotide repeat expansions that form at least a secondary structure within said mRNA. The binding between the artificial RNA of the invention to the pathological target mRNA causes the disruption of the secondary structure of the target mRNA. In other words, the present invention relates to artificial RNAs suitable for disrupting by hybridization of at least one secondary structure comprised in the pathological target mRNA, wherein said secondary structure is formed by pathological nucleotide repeat expansions.

By "**disrupting by hybridization of at least one secondary structure comprised in the pathological target mRNA**" it is understood, in the context of the present invention, that the secondary structure of a pathological target mRNA is altered when a region (called herein the hybridization region) of the artificial RNA of the invention hybridizes with a region (called herein a target hybridization region) of the secondary structure of the pathological target mRNA. The disruption by hybridization causes the disappearance of said secondary structure.

By "**pathological nucleotide repeat expansions**" is meant herein a region in the pathological target mRNA that comprises a nucleotide sequence that is repeated in a number of times that is higher than the number of repetitions found for the same nucleotide sequence in the same mRNA of a healthy cell. A pathological target mRNA having said pathological nucleotide repeat expansion causes a pathological phenotype, or a risk of having a pathological phenotype, in the individual carrying it. The target mRNA is thus a pathological target mRNA, i.e., it is associated to and/or is causing a disease, and the artificial RNA of the invention is capable of inhibiting the pathological function of the target mRNA y disrupting the secondary structure present in it. In the context of the present invention, the term "**pathological**" refers to a phenotype or genotype that causes a disease, or a risk of having it.

The expression "**causing a disease**" refers herein to producing a disease, or being the reason or the trigger of a disease. The expression "**a pathological target mRNA that causes a risk of having a pathological phenotype**" or "**a pathological target mRNA that causes a risk of having a certain disease**" refers to a pathological target mRNA that comprises pathological nucleotide repeat expansions, so that the individual carrying it (i.e., the individual whose cells carry said pathological target mRNA), has a higher likelihood or probability of presenting a disease than a control individual. For example, a pathological target DMPK mRNA comprising more than 38 CUG trinucleotide repeats in its 3'UTR causes Steinert disease or a risk of having Steiner disease in the individual where this target DMPK mRNA is identified. It is understood that "**a risk of having a pathological phenotype**" or "**a risk of having a certain disease**" is meant an increased risk of having said phenotype or disease as compared to a control subject. The increased risk may be relative or absolute and may be expressed qualitatively or quantitatively. For example, an increased risk may be expressed as simply determining the number of pathological expansion repeats in a pathological target mRNA and classifying the individual carrying said target mRNA in an "increased risk" category, based upon previous population studies and control samples. As used herein, examples of expressions of an increased risk include but are not limited to, odds, probability, odds ratio, p-values, attributable risk, relative frequency, positive predictive value, negative predictive value, and relative risk.

Hence, the artificial RNA of the invention is capable of binding or hybridizing to a pathological target mRNA, wherein the pathological target mRNA is characterized by comprising:
(a) pathological nucleotide repeat expansions, and
(b) at least one secondary structure,
wherein the artificial RNA of the invention comprises at least one hybridization region that hybridizes to at least a portion of the secondary structure, thereby disrupting by hybridization said secondary structure of the target mRNA.

### (a) pathological nucleotide repeat expansions

Pathological or abnormal expansions of simple sequence repeats are characteristic of a series of diseases most of which primarily affect the nervous system. Trinucleotide repeat expansions causing diseases were discovered first and remain the most frequent. However, tetra-, penta-, hexa- and even dodeca-nucleotide repeat expansions have also been identified as the cause of human disease. In the context of the present invention, the target mRNA is characterized by having pathological nucleotide repeat expansions, which are nucleotide repeat expansions that cause a disease or a pathological phenotype, or a risk of having it. Preferably, the sequence of nucleotides that is repeated in the pathological target mRNA is a sequence of between 3 to 5 nucleotides (trinucleotide, tetranucleotide, and pentanucleotide nucleotide repeat expansions). However, the sequence of nucleotides that is repeated may be longer, such as 6, 7, 8, 9, 10, 11, or 12 or more nucleotide repeat expansions.

The pathological nucleotide repeat expansions comprised in the pathological target mRNA and that cause a disease or a pathological phenotype, or a risk of having it, can also be represented as (N₁N₂Nₖ)a nucleotide repeats, wherein a is 25 or more, preferably 50 or more, and wherein k represents 1, 2, 3, 4, 5, 6, 7, 8, 9 or more nucleotides, preferably between 1 and 3 nucleotides. Preferably, the pathological nucleotide repeat expansions comprised in the target mRNA are pathological trinucleotide expansions, also represented herein as (N₁N₂N₃)a, wherein a is 25 or more, preferably 50 or more. Preferably, the pathological nucleotide repeat expansions comprised in the target mRNA are pathological tretranucleotide expansions, also represented herein as (N₁N₂N₃N₄)a, wherein a is 25 or more, preferably 50 or more. Preferably, the pathological nucleotide repeat expansions comprised in the target mRNA are pathological pentanucleotide expansions, also represented herein as (N₁N₂N₃N₄N₅)a, wherein a is 25 or more, preferably 50 or more.

In an embodiment, the sequence of nucleotides, preferably 3-5 nucleotides, is repeated in the pathological target mRNA at least 25 times or more, preferably at least 50 times or more. Thus, the nucleotide repeat expansions may range from more than 25 repeats, more than 50 repeats, such as between 50-1000 repeats, depending on the subject and the disease.

Preferably, the pathological nucleotide repeat expansions are located in the 5' UTR, in the CDS region, or in the 3' UTR of the pathological target mRNA.

### (b) at least one secondary structure

As explained above, the pathological target mRNA is also characterized by comprising at least a secondary structure. Said secondary structure is formed as a result of the pathological nucleotide repeat expansions that self-hybridize. Thus, thus the secondary structure overlaps, partially or fully, with the pathological nucleotide repeat expansions of (a).

The artificial RNAs of the invention can hybridize, preferably completely hybridize, with at least a region of the at least one secondary structure within the pathological target mRNA. In doing so, the artificial RNAs of the present invention disrupt the secondary structure within the pathological target mRNA.

In an embodiment, the artificial RNA of the invention is capable of hybridizing to a pathological target mRNA, wherein the pathological target mRNA is characterized by comprising:
(a) pathological nucleotide repeat expansions, as explained above, and
(b) at least one secondary structure comprising:
   (i) a hairpin loop formed by all or some of the pathological nucleotide repeat expansions, and
   (ii) at least one target hybridization region,
wherein the artificial RNA of the invention comprises at least one hybridization region that hybridizes, preferably completely hybridizes, with the at least one target hybridization region of the at least one secondary structure, thereby disrupting by hybridization said secondary structure of the pathological target mRNA.

In the context of the present invention a "**secondary structure**" within a pathological target mRNA molecule comprises a stem loop (or hairpin loop), preferably preceded or followed by a stretch of unpaired nucleotides, (external loop, bulge or internal loop or multiloop) and at least one target hybridization region. In an embodiment, the secondary structure is a region of the target mRNA comprises (i) at least a hairpin loop formed by all or some of the pathological nucleotide repeat expansions and that is preceded or followed by a region of unpaired nucleotides. The hairpin loop may be comprised in a G-quadruplex structure, or a duplex structure, or any other secondary structure.

The secondary structure also comprises (ii) one or more of the so-called "**target hybridization region(s)**". Said target hybridization region may overlap, fully or partially, with the hairpin loop of (i). Preferably, the target hybridization region overlaps partially with the hairpin loop of (i). In the context of the present invention, a "**target hybridization region**" is a region comprised in the secondary structure of the pathological target mRNA, and it is composed of a single stranded region preceded or followed by a double-stranded region. The target hybridization region is a region that, upon hybridization of the artificial RNA of the invention, yields the disruption of the secondary structure to which it belongs to. Thus, the secondary structure in the target RNA molecule is also called herein a "**target disruption structure**", since it is a structure that will be disrupted by hybridization between the hybridization region of the artificial RNA of the invention and the target hybridization region comprised in the secondary structure of the pathological target RNA.

The target hybridization regions are regions (i.e., a sequence of nucleotides) which comprises a single-stranded region of at least 2 nucleotides preceded or followed by a double-stranded region. The length of the target hybridization region is not limited as long as it comprises a single-stranded region of at least 2 nucleotides preceded or followed by a double-stranded region and is able to hybridize, preferably completely hybridize, with the at least one hybridization region of the artificial RNA of the invention. Preferably, the single-stranded region of the target hybridization region comprises 3 nucleotides or more, such as 3, 4, 5, 10, 15 nucleotides or more. Preferably the double-stranded region of the target hybridization region comprises 5 nucleotides or more, such as 5, 7, 10, 15, 20, 25 nucleotides or more. Preferably, the target hybridization region comprised in the secondary structure of the pathological target mRNA comprises a single-stranded region of at least 2 nucleotides, preferably 3 nucleotides or more, preceded or followed by a double-stranded region of at least 5 nucleotides, preferably 10 nucleotides or more.

The artificial RNAs of the present invention hybridize with the secondary structure through the so-called "**hybridization regions**". In the context of the present invention a "**hybridization region**" is a ribonucleotide sequence comprised in the artificial RNA of the invention that is capable of hybridizing, preferably completely, with at least one target hybridization region comprised in the secondary structure of a pathological target mRNA molecule. When the hybridization region of an artificial RNA of the invention hybridizes with the target hybridization region of a pathological target mRNA, structural changes occur in the pathological target mRNA that modulate its function. In other words, the artificial RNAs of the invention comprise at least one hybridization region, which is a region of the artificial RNA of the invention that is able to hybridize, preferably completely hybridize, with the one or more target hybridization regions comprised in the at least one secondary structure of the pathological target mRNA. The artificial RNA of the invention may have one, two, three, four, five, or more hybridization regions.

**Hybridization** (or hybridisation) is a phenomenon in which two RNA molecules anneal to each other via base pairing interactions. In the context of this invention only canonical base pairings are considered (C-G, A-U and G-U). By "**complete hybridization**" it is understood that all of the nucleotides of the hybridization region of the artificial RNA anneal with all of the nucleotides of the target hybridization region.

In an embodiment, the hybridization region of the artificial RNA of the invention completely hybridizes with at least one target hybridization region comprised in the at least one secondary structure within the pathological target mRNA. When the hybridization region of the artificial RNA of the invention completely hybridizes with the target hybridization region of the secondary structure, the energy of the hybridization between the hybridization region and the target hybridization region is more negative than the energy of the secondary structure. When this happens, the secondary structure is disrupted, i.e., the secondary structure of the pathological target mRNA is altered. Hence, the pathological functionality of the target mRNA is also altered: the pathological functionality of the target mRNA has been modulated by the artificial RNA of the present invention.

The number of nucleotides of the hybridization region is not limited as long as it is able to hybridize, preferably completely hybridize, with the at least one target hybridization region, although preferably it has the same length as the target hybridization region comprised in the secondary structure and, when they hybridize, the secondary structure is disrupted. In a preferred embodiment, the at least one hybridization region has a total of between 7 and 100 nucleotides, more preferably between 10 and 50 nucleotides, even more preferably between 15 and 35 nucleotides, such as between 15 and 25 nucleotides.

In the preferred embodiment where the hybridization region of the artificial RNA of the invention completely hybridizes with the at least one target hybridization region comprised in the at least one secondary structure of the pathological target mRNA, the at least one hybridization region of the artificial RNA of the present invention has exactly the same number of nucleotides than the at least one target hybridization region with which it hybridizes. Consequently, the at least one hybridization region of the artificial RNA of the invention has a first region of a certain number of nucleotides which preferably completely hybridizes with the single-stranded region of the target hybridization region, and a second region of a certain number of nucleotides which preferably completely hybridizes with the double-stranded region of the target hybridization region.

In other words, in a first step, the single-stranded region of the target hybridization region anneals with certain nucleotides of the hybridization region of the artificial RNA of the invention. In a second step, the double-stranded region of the target hybridization region, which precedes or follows the single-stranded region, is disrupted, and new interactions are formed between one strand of the double-stranded region of the target hybridization region and certain nucleotides of the hybridization region of the artificial RNA of the invention, which anneal to each other. The disruption of the double-stranded region of the target hybridization region occurs when the energy of the hybridization between the at least one hybridization region and the at least one target hybridization region is more negative (more favourable) than the energy of the secondary structure.

As a result of the hybridization between the artificial RNA of the invention and the pathological target mRNA, at least the double-stranded region of the target hybridization region is disrupted and, hence, at least one secondary structure of the target mRNA is also disrupted. Hence, the structure of the secondary structure of the pathological target mRNA changes, at least, completely for the overlap between the secondary structure and the target hybridization contained in it. Therefore, the secondary structure of the pathological target mRNA is changed and, thus, its functionality is also altered.

Consequently, the at least one hybridization region comprised in the artificial RNA of the invention is further characterized because, when hybridizing with the at least one target hybridization region, the energy of the hybridization between the at least one hybridization region and the at least one target hybridization region is more negative than the energy of the secondary structure comprised in the pathological target mRNA, thereby disrupting said secondary structure. As will be explained in detail below, the skilled person is able to design predict the energy of the hybridization between the at least one hybridization region and the at least one target hybridization region and the energy of the secondary structure.

The artificial RNA of the invention can be of any length as long as it comprises at least one hybridization region as described in the present invention and is suitable for disrupting by hybridization one or more secondary structures of one or more target mRNA. In an embodiment, the artificial RNA of the invention comprises between 10-50, 10-40, 10-30, preferably 15-30, nucleotides in length.

### The pathological target mRNA

As explained above, some diseases, preferably human diseases, are caused by the presence of pathological mRNAs in the cells. Said mRNAs are characterized by having pathological nucleotide repeat expansion that form secondary structures by self-hybridization and affect the normal function of the mRNA. It is noted that a given mRNA is only considered pathological when the number of nucleotide repeats exceeds the number of nucleotide repeats in the same mRNA in a healthy individual, and when this nucleotide repeat expansions causes a phenotype of disease or a risk thereof. That is, a pathological target mRNA is a target mRNA that comprises pathological nucleotide repeat expansions. In the context of the present invention, "pathological target mRNA" and "target mRNA" are considered synonymous and are used interchangeably.

Thus, the pathological target mRNA is considered pathological when it causes a phenotype of a disease, or a risk of having a phenotype of a disease, due to the presence of more nucleotide repeats than those present in the corresponding mRNA in cells of a healthy individual. In other words, the target mRNA is characterized by having a pathological (disease causing) allele. Of course, the pathological target mRNA is generated from the transcription of a pathological gene, being said pathological gene a region of the DNA comprising pathological nucleotide repeat expansions. As explained above, the pathological target mRNA is characterized by causing a disease or a risk of having a disease, and by comprising:
(a) pathological nucleotide repeat expansions, and
(b) at least one secondary structure formed by the pathological nucleotide repeat expansions.

In an embodiment, the pathological target mRNA causes **Steinert disease** or a risk of having Steinert disease. Steinert's disease, or myotonic dystrophy type 1 (DM1), is caused by a repeated expansion of CTG of more than 37 units in the 3'UTR of the gene encoding protein myotonic dystrophy protein kinase (DMPK). In healthy individuals, the expansion of these trinucleotides ranges from 5 to 37 repeats, while in people with Steinert or at risk of having Steinert, it expands when more than 37 repeats are found, preferably more than 50, more preferably more than 500, repeats are found. Thus, a DMPK mRNA comprising in its 3'UTR more than 37, preferably more than 50, CUG repeats causes Steinert disease or a risk of having Steinert disease, and thus it is called herein a pathological target DMPK mRNA.

Therefore, in an embodiment, the artificial RNA of the invention is capable of hybridizing to a pathological target DMPK mRNA, wherein the pathological target DMPK mRNA is characterized by comprising in its 3'UTR:
(a) (CUG)a trinucleotide repeats, wherein a is 38 or more, preferably 50 or more, and
(b) at least one secondary structure comprising:
   (i) a hairpin loop formed by at least part of the repeated sequence of nucleotides of (a), and
   (ii) at least one **target hybridization region** which comprises a single-stranded region of at least 2 nucleotides, preferably 3 nucleotides or more, preceded or followed by a double-stranded region of at least 5 nucleotides, preferably 10 nucleotides or more; and
wherein the artificial RNA molecule comprises at least one **hybridization region** that hybridize, preferably completely hybridize, with the at least one target hybridization region, thereby disrupting by hybridization the secondary structure of the pathological target DMPK mRNA.

The Examples shown below describe the identification of three target hybridization regions within the pathological target DMPK mRNA, and the design of artificial RNAs of the invention capable of disrupting the secondary structure of said target mRNA.

In an embodiment, the target hybridization region comprised in the secondary structure of the pathological target DMPK mRNA comprises or consists of SEQ ID NOs: 76, 77, 78 or 79.

Tables 2 and 4 show (see Examples section), in the right column, the several artificial RNA of the invention designed against the target hybridization region of SEQ ID NO: 76, 77, 78 or 79. Thus, in a preferred embodiment, the artificial RNA of the invention that is capable of disrupting by hybridization a secondary structured formed by the CUG trinucleotide expansions present in a pathological target DMPK mRNA comprises or consist of SEQ ID NOs: 9-56, or SEQ ID NOs: 73, 74 and 75, or a sequence that has at least 75%, 80%, 85%, 90%, 91%, 92%, 93%, 94%, 95%, 96%, 97%, 98%, or 100% sequence identity to SEQ ID NO: 9-56 or 73-75, respectively.

In an embodiment, the target hybridization region comprises SEQ ID NO: 76, and the artificial RNA of the invention that is capable of disrupting by hybridization a secondary structure formed by the CUG trinucleotide expansions present in a pathological target DMPK mRNA comprises or consist of SEQ ID NOs: 9, 10, 11, 12, 13, 14, 15, 16, 17, 18, 19, 20, 39, 41, 43, 51, 54, and 73 or a sequence that has at least 75%, 80%, 85%, 90%, 91%, 92%, 93%, 94%, 95%, 96%, 97%, 98%, or 100% sequence identity to SEQ ID NOs: 9, 10, 11, 12, 13, 14, 15, 16, 17, 18, 19, 20, 39, 41, 43, 51, 54, and 73, respectively.

In an embodiment, the target hybridization region comprises SEQ ID NO: 77, and the artificial RNA of the invention that is capable of disrupting by hybridization a secondary structure formed by the CUG trinucleotide expansions present in a pathological target DMPK mRNA comprises or consist of SEQ ID NOs: 21, 22, 23, 24, 25, 26, 27, 28, 29, 30, 31, 32, 40, 42, 44, 45, 47, 49, 52, 55, and 74 or a sequence that has at least 75%, 80%, 85%, 90%, 91%, 92%, 93%, 94%, 95%, 96%, 97%, 98%, or 100% sequence identity to SEQ ID NOs: 21, 22, 23, 24, 25, 26, 27, 28, 29, 30, 31, 32, 40, 42, 44, 45, 47, 49, 52, 55, and 74, respectively.

In an embodiment, the target hybridization region comprises SEQ ID NO: 78, and the artificial RNA of the invention that is capable of disrupting by hybridization a secondary structure formed by the CUG trinucleotide expansions present in a pathological target DMPK mRNA comprises or consist of SEQ ID NOs: 33, 34, 35, 36, 37, 38, 46, 48, 50, 53, 56, and 75 or a sequence that has at least 75%, 80%, 85%, 90%, 91%, 92%, 93%, 94%, 95%, 96%, 97%, 98%, or 100% sequence identity to : 33, 34, 35, 36, 37, 38, 46, 48, 50, 53, 56, and 75, respectively.

In an embodiment, the pathological target mRNA causes **amyotrophic lateral sclerosis (ALS) and related disorders of the ALS/frontotemporal dementia (FTD) spectrum,** or a risk of having ALS/FTD disorders. ALS/FTD is caused by a repeated expansion of GGGGCC hexanucleotide in the gene C9ORF72. In healthy individuals, the expansion of this hexanucleotide repeat ranges from 30 repeats or less, while in people with ALS/FTD phenotype or a risk of having said phenotype, more than 30, preferably more than 60, more preferably more than 100, GGGGCC repeats are found. Thus, a C9ORF72 mRNA comprising in its intron 1 more than 30, preferably more than 100, GGGGCC repeats causes ALS/FTLD disease or a risk of having ALS/FTD disease, and thus it is called herein a pathological target C9ORF72 mRNA.

Therefore, in an embodiment, the artificial RNA of the invention is capable of hybridizing to a pathological target C9ORF72 mRNA, wherein the pathological target C9ORF72 mRNA is characterized by comprising in its intron 1:
(a) (GGGGCC)a hexanucleotide repeats, wherein a is 30 or more, preferably 60 or more, and
(b) at least one secondary structure comprising:
   (i) a hairpin loop formed by at least part of the repeated sequence of nucleotides of (a), and
   (ii) at least one **target hybridization region** which comprises a single-stranded region of at least 2 nucleotides, preferably 3 nucleotides or more, preceded or followed by a double-stranded region of at least 5 nucleotides, preferably 10 nucleotides or more; and
wherein the artificial RNA molecule comprises at least one **hybridization region** that hybridize, preferably completely hybridize, with the at least one target hybridization region, thereby disrupting by hybridization the secondary structure of the pathological target C9ORF72 mRNA.

In an embodiment, the pathological target mRNA causes **Spinocerebellar ataxia type 8 (SCA8)** or a risk of having SCA8. SCA8 is caused by repeat expansion mutation present in the genes ATXN8 and ATXN8OS that are bidirectionally transcribed. When an individual has SCA8, the mRNA of each gene (ATXN8 mRNA and ATXN8OS mRNA) comprise pathological repeat expansions: in the case of ATXN8 mRNA the pathological repeat expansion is placed in exon 1 and is a CAG trinucleotide repeat expansion; while and in the case of ATXN8OS mRNA the pathological repeat expansion is placed in the 5' UTR and is a CTG trinucleotide repeat expansion. Said expansions are also usually flanked by TAG repeats in ATXN8 mRNA and CTA repeats in ATXN8OS mRNA. In healthy individuals, the expansion of these trinucleotide repeat ranges from 15 to 50 repeats, while in people with SCA8 or at risk of having SCA8, more than 51 repeats are found, preferably when more than 80 repeats are found, more preferably over 1300 CAG/CTG repeats are found. Thus, ATXN8 and ATXN8OS mRNAs comprising in its exon 1 and 5'UTR, respectively, more than 51, preferably more than 80, CAG and CTG repeats, respectively, cause SCA8 disease or a risk of having SCA8 disease, and thus they are called herein a pathological target ATXN8 and ATXN8OS mRNAs, respectively.

Therefore, in an embodiment, the artificial RNA of the invention is capable of hybridizing to a pathological target ATXN8 mRNA, wherein the pathological target ATXN8 mRNA is characterized by comprising in its exon 1:
(a) (CAG)a trinucleotide repeats, wherein a is 51 or more, preferably 80 or more, and
(b) at least one secondary structure comprising:
   (i) a hairpin loop formed by at least part of the repeated sequence of nucleotides of (a), and
   (ii) at least one **target hybridization region** which comprises a single-stranded region of at least 2 nucleotides, preferably 3 nucleotides or more, preceded or followed by a double-stranded region of at least 5 nucleotides, preferably 10 nucleotides or more; and
wherein the artificial RNA molecule comprises at least one **hybridization region** that hybridize, preferably completely hybridize, with the at least one target hybridization region, thereby disrupting by hybridization the secondary structure of the pathological target ATXN8 mRNA.

Also, in another embodiment, the artificial RNA of the invention is capable of hybridizing to a pathological target ATXN8OS mRNA, wherein the pathological target ATXN8OS mRNA is characterized by comprising in its 5'UTR:
(a) (CTG)a trinucleotide repeats, wherein a is 51 or more, preferably 80 or more, and
(b) at least one secondary structure comprising:
   (i) a hairpin loop formed by at least part of the repeated sequence of nucleotides of (a), and
   (ii) at least one **target hybridization region** which comprises a single-stranded region of at least 2 nucleotides, preferably 3 nucleotides or more, preceded or followed by a double-stranded region of at least 5 nucleotides, preferably 10 nucleotides or more; and
wherein the artificial RNA molecule comprises at least one **hybridization region** that hybridize, preferably completely hybridize, with the at least one target hybridization region, thereby disrupting by hybridization the secondary structure of the pathological target ATXN8OS mRNA.

In an embodiment, the pathological target mRNA causes **FMR1 disorders,** or a risk of having a FMR1 disorder. FMR1 disorders include fragile X syndrome (FXS), fragile X-associated tremor/ataxia syndrome (FXTAS), and fragile X-associated primary ovarian insufficiency (FXPOI). FMR1 disorders are caused by a repeated expansion of CGG of more than 55 units in the 5'UTR of the FMR1. In healthy individuals, the expansion of these trinucleotides ranges from 5 to 54 repeats, while in people with an FMR1 disorder or at risk of having an FMR1 disorder, it expands when more than 55 repeats are found, preferably more than 100, more preferably more than 200, repeats are found. Thus, a FMR1 mRNA comprising in its 5'UTR more than 55, preferably more than 100, CGG repeats causes FMR1 disorders or a risk of having FMR1 disorders, and thus it is called herein a pathological target FMR1 mRNA.

Therefore, in an embodiment, the artificial RNA of the invention is capable of hybridizing to a pathological target FMR1 mRNA, wherein the pathological target FMR1 mRNA is characterized by comprising in its 5'UTR:
(a) (CGG)a trinucleotide repeats, wherein a is 55 or more, preferably 100 or more, and
(b) at least one secondary structure comprising:
   (i) a hairpin loop formed by at least part of the repeated sequence of nucleotides of (a), and
   (ii) at least one **target hybridization region** which comprises a single-stranded region of at least 2 nucleotides, preferably 3 nucleotides or more, preceded or followed by a double-stranded region of at least 5 nucleotides, preferably 10 nucleotides or more; and
wherein the artificial RNA molecule comprises at least one **hybridization region** that hybridize, preferably completely hybridize, with the at least one target hybridization region, thereby disrupting by hybridization the secondary structure of the pathological target FMR1 mRNA.

In an embodiment, the pathological FMR1 target mRNA causes FXPOI and/or FXTAS, or a risk of having FXPOI and/or FXTAS. FXPOI and FXTAS are caused by 55-200 repeat expansion of CGG trinucleotide in the 5' UTR of FMR1 gene. In healthy individuals, the expansion of this trinucleotide repeat ranges from 5 to 54 repeats, while in people with FXPOI and/or FXTAS, or at risk of having FXPOI and/or FXTAS, between 55-200 repeats are found, preferably between 100-200 repeats. Thus, a FMR1 mRNA comprising in its 5'UTR between 55-200, preferably between 100-200, CGG repeats causes FXPOI and/or FXTAS disease, or a risk of having FXPOI and/or FXTAS disease, and thus it is called herein a pathological FXPOI and/or FXTAS target FMR1 mRNA.

Therefore, in an embodiment, the artificial RNA of the invention is capable of hybridizing to a pathological FXPOI and/or FXTAS target FMR1 mRNA, wherein the pathological FXPOI and/or FXTAS target FMR1 mRNA is characterized by comprising in its 5'UTR:
(a) (CGG)a trinucleotide repeats, wherein a is between 55-200, preferably 100-200, and
(b) at least one secondary structure comprising:
   (i) a hairpin loop formed by at least part of the repeated sequence of nucleotides of (a), and
   (ii) at least one **target hybridization region** which comprises a single-stranded region of at least 2 nucleotides, preferably 3 nucleotides or more, preceded or followed by a double-stranded region of at least 5 nucleotides, preferably 10 nucleotides or more; and
wherein the artificial RNA molecule comprises at least one **hybridization region** that hybridize, preferably completely hybridize, with the at least one target hybridization region, thereby disrupting by hybridization the secondary structure of the pathological FXPOI and/or FXTAS target FMR1 mRNA.

In an embodiment, the pathological target mRNA causes **Huntington's disease (HD)** or a risk of having HD. HD is caused by a repeated expansion of CAG trinucleotide in exon 1 of the HTT gene. In healthy individuals, the expansion of this trinucleotide repeat ranges from 9 to 37 repeats, while in people with HD or at risk of having HD, more than 38 repeats are found, preferably more than 50 repeats are found, more preferably between 37-120 CAG repeats are found. Thus, a HTT mRNA comprising in its exon 1 more than 38, preferably more than 50, CAG repeats causes HD disease or a risk of having HD disease, and thus it is called herein a pathological target HTT mRNA.

Therefore, in an embodiment, the artificial RNA of the invention is capable of hybridising to a pathological target HTT mRNA, wherein the pathological target HTT mRNA is characterised by comprising in its exon 1:
(a) (CAG)a trinucleotide repeats, wherein a is 38 or more, preferably 50 or more, and
(b) at least one secondary structure comprising:
   (i) a hairpin loop formed by at least part of the repeated sequence of nucleotides of (a), and
   (ii) at least one **target hybridization region** which comprises a single-stranded region of at least 2 nucleotides, preferably 3 nucleotides or more, preceded or followed by a double-stranded region of at least 5 nucleotides, preferably 10 nucleotides or more; and
wherein the artificial RNA molecule comprises at least one **hybridization region** that hybridize, preferably completely hybridize, with the at least one target hybridization region, thereby disrupting by hybridization the secondary structure of the pathological target HTT mRNA.

### Mode of action and design of the artificial RNA of the invention

As explained above, the target mRNA is a pathological mRNA, i.e., it is associated and/or is causing a disease, and the artificial RNA of the invention is capable of inhibiting the pathological function of the target mRNA. This inhibition is caused by the hybridization between the hybridization region of the artificial RNA of the invention and the target hybridization region of the secondary structure of the pathological target mRNA.

Thus, the artificial RNAs of the invention are designed so that they hybridize with selected regions (target hybridization regions) present in secondary structure of a pathological target mRNA that has a pathological phenotype characterized by nucleotide repeats expansion. However, the mechanism of action is not just the hybridization between the hybridization regions of the artificial RNA and the target hybridization regions of the secondary structure, but the structural changes triggered upon this hybridization will alter the secondary structure of the target mRNA, ultimately affecting its function. This is because the binding of the designed artificial RNAs to the secondary structure in the pathological target mRNA leads to the unfolding of the secondary structure (changes in the secondary structure) and consequently to the reduction or even inhibition of the pathological function of said pathological target mRNA.

Diseases caused by the presence pathological target mRNAs (i.e., caused by nucleotide repeat expansion) are known in the art. For a given pathological target mRNA, the skilled person is able to identify the at least one secondary structure caused by the nucleotide repeat expansion, since these are regions of RNA which comprise at least a portion of a hairpin loop preceded or followed by a region of unpaired nucleotides. There are many RNA structure prediction software available to the skilled person and that predict the secondary structure of a given RNA sequence. For instance, we refer to Mathews, D. H., et al. "RNA secondary structure prediction." Current protocols in nucleic acid chemistry vol. Chapter 11 (2007): Unit 11.2. doi:10.1002/0471142700.nc1102s28.

Since the secondary structure of the target mRNA is a consequence of the presence of the pathological nucleotide repeat expansions, it can also be predicted either computationally or with experimental methods. Computationally, RNA secondary structure can be predicted from one or several nucleic acid sequences using tools publicly available to the skilled person. For example, the structure and the energy of a given sequence can be calculated using:
- **NUPACK** (http://www.nupack.org/design/new, see also B. R. Wolfe, N. J. Porubsky, J. N. Zadeh, R. M. Dirks, and N. A. Pierce, "Constrained multistate sequence design for nucleic acid reaction pathway engineering", J Am Chem Soc, 139:3134-3144, 2017; B. R. Wolfe and N. A. Pierce, "Sequence design for a test tube of interacting nucleic acid strands", ACS Synth Biol, 4:1086-1100, 2015; J. N. Zadeh, B. R. Wolfe, and N. A. Pierce, "Nucleic acid sequence design via efficient ensemble defect optimization" J Comput Chem, 32:439-452, 2011; and R. M. Dirks, M. Lin, E. Winfree, and N. A. Pierce, "Paradigms for computational nucleic acid design" Nucl Acids Res, 32:1392-1403, 2004.),
- **RNAcofold** (Lorenz, Ronny and Bernhart, Stephan H. and Höner zu Siederdissen, Christian and Tafer, Hakim and Flamm, Christoph and Stadler, Peter F. and Hofacker, Ivo L., ViennaRNA Package 2.0, Algorithms for Molecular Biology, 6:1 26, 2011, doi:10.1186/1748-7188-6-26; Reuter, J. S., & Mathews, D. H. (2010). RNAstructure: software for RNA secondary structure prediction and analysis. BMC Bioinformatics. 11,129, Mathews, D. H. et al., "Predicting oligonucleotide affinity to nucleic acid targets", RNA, 1999 5: 1458-1469),
- **RNAstructure** (https://rna.urmc.rochester.edu/RNAstructure.html),
- **Mfold** (http://unafold.rna.albany.edu/?q=mfold) M. Zuker, "Mfold web server for nucleic acid folding and hybridization prediction", Nucleic Acids Res. 31 (13), 3406-3415, 2003), or
- **Vienna package** (http://rna.tbi.univie.ac.at/) Lorenz, Ronny and Bernhart, Stephan H. and Höner zu Siederdissen, Christian and Tafer, Hakim and Flamm, Christoph and Stadler, Peter F. and Hofacker, Ivo L., ViennaRNA Package 2.0, Algorithms for Molecular Biology, 6:1 26, 2011, doi:10.1186/1748-7188-6-26).

Experimental methods to observe secondary structures include topological investigations using gel-shift assay, CD spectroscopy, NMR spectroscopy, or chemical probe experiments like SHAPE.

In addition, the skilled person is able to identify one or more target hybridization regions within the at least one secondary structure, because the target hybridization region is comprised in the at least one secondary structure and comprises a single-stranded region of at least 2 nucleotides preceded or followed by a double-stranded region, as defined above.

Once a target hybridization region for a given target mRNA is identified, the skilled person is able to design one or more hybridization regions which would hybridize, preferably completely hybridize, with the target hybridization region. This can be done with publicly available software for the design of RNA sequences which would completely hybridize with a given RNA sequence, e.g.:
- **RNAiFold** (https://bioinformatics.bc.edu/clotelab/RNAiFold/, see also Juan Antonio Garcia-Martin, Peter Clote, Ivan Dotu, "RNAiFold: A constraint programming algorithm for RNA inverse folding and molecular design, J Bioinform Comput Biol 11 (2): 1350001, 2013; and Garcia-Martin JA, Dotu I, Clote P., "RNAiFold 2.0 A web server and software to design custom and Rfam-based RNA molecules", Nucleic Acids Research Web Server issue, 2015, doi: 10.1093/nar/gkv460, "RNAiFold 2.0 A web server and software to design custom and Rfam-based RNA molecules"), or
- **MoiRNAiFold** (https://moiraibiodesign.com/design/).

The skilled person is thus capable of calculating whether the energy of the hybridization between the designed hybridization regions and the target hybridization region is more negative than the energy of the secondary structure, and thus whether the artificial RNA candidate will disrupt the secondary structure.

Preferably, the energy of the hybridization between the at least one hybridization region and the at least one target hybridization region and the energy of secondary structure is calculated with **RNAcofold** software, with the settings as described in Mathews, D. H., et al., "Predicting oligonucleotide affinity to nucleic acid targets", RNA, 1999 5: 1458-1469. It is however noted that such calculation shall be unnecessary when RNA inverse folding tools, such as NUPACK, RNAifold, or MoiRNAiFold, are used for the identification of potential candidates of the hybridization regions of the artificial RNA of the present invention characterized by having an energy of the hybridization between the at least one hybridization region and the at least one target hybridization region more negative (more favourable) than the energy of the secondary structure.

The hybridization regions of the artificial RNA of the present invention characterized by having an energy of the hybridization between the at least one hybridization region and the at least one target hybridization region more negative (more favourable) than the energy of the secondary structure, can be easily designed with **RNAiFold** (http://bioinformatics.bc.edu/clotelab/RNAiFold/) or its recent extension **MoiRNAiFold** (https://moiraibiodesign.com/design/), and subsequent validated with **NUPACK** (http://www.nupack.org/design/new), or **RNAcofold.**

For instance, in RNAiFold (and MoiRNAiFold), given any specific target disruption structure, such as (UUGUAGCCGGGAAUGCUGCUGCUGCUGCUGCUGCUGCUGCUGCUGCUGCUGCUGC UGCUGCUGCUGCUGCUGCUGGGGGGAUCACAGACCAU) and its target hybridization region (**UUGUAGCCGGGAAUGCUGCUGCUGCUGCUG**), one can use the following input file:
#RNAscdstr ((((((((((((((((((((((((((((((&)))))))))))))))))))))))))))))),,,,,,,,,,,,,,,,,,,,,,,,,,,,,,,,,,,,,,,,,,,,,,,,,,,,,,,,,,, #RNAseqcon

NNNNNNNNNNNNNNNNNNNNNNNNNNNNNN&UUGUAGCCGGGAAUGCUGCUGCUGC
UGCUGCUGCUGCUGCUGCUGCUGCUGCUGCUGCUGCUGCUGCUGCUGCUGGGGGG
AUCACAGACCAU

Wherein the input of ((((((((((((((((((((((((((((((&)))))))))))))))))))))))))))))),,,,,,,,,,,,,,,,,,,,,,,,,,,,,,,,,,,,,,,,,,,,,,,,,,,,,,,,,,, corresponds to the well-established dot bracket notation (see, e.g. RNAlib-2.4.18: RNA Structure Notations (univie.ac.at), with the extension of ',' representing "either base paired or unpaired" and wherein the input NNNNNNNNNNNNNNNNNNNNNNNNNNNNNN&UUGUAGCCGGGAAUGCUGCUGCUGC UGCUGCUGCUGCUGCUGCUGCUGCUGCUGCUGCUGCUGCUGCUGCUGCUGGGGGG AUCACAGACCAU indicates that every "N" is a nucleotide (any nucleotide A,C,G or U) corresponding with "(" above and that has to hybridize with the corresponding ")" indicated above. That is, the RNAiFold (and MoiRNAiFold) should return a solution (or multiple ones) of potential hybridization regions that can indeed disrupt the target disruption structure UUGUAGCCGGGAAUGCUGCUGCUGCUGCUGCUGCUGCUGCUGCUGCUGCUGCUGC UGCUGCUGCUGCUGCUGCUGGGGGGAUCACAGACCAU upon complete hybridization to the target hybridization region UUGUAGCCGGGAAUGCUGCUGCUGCUGCUG.

In this case, a sample set of 10 solutions return by the RNA inverse folding tool was the following:

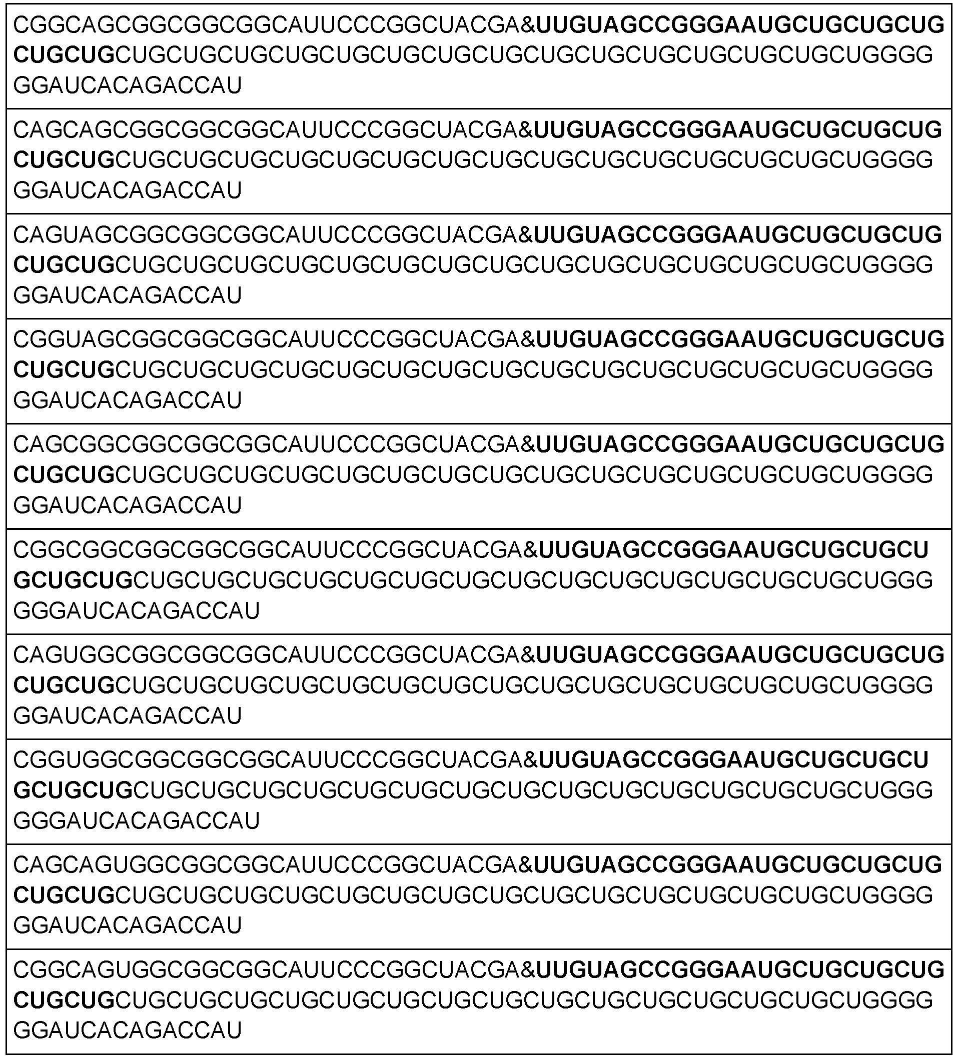

from which it follows first, that the target disruption structure can be disrupted via hybridization and second, that the hybridization regions (in bold) can be used to construct an artificial RNA of the invention, comprising one or more of them. If a circRNA of the invention (see below) wants to be designed, the skilled person only needs to separate these regions with random polynucleotide regions.

Thus, provided these input instructions, if the software tool returns a solution (or multiple ones) it means that the target disruption structure can indeed be disrupted by hybridization. From the set of solutions it follows the generation of the circRNA of this invention (see below): by taking any number of these solutions (hybridization regions) and separating them by random nucleotides regions.

Lastly, the hybridization between the hybridization region of the artificial RNA and the target hybridization region of the at least one secondary structure of the target mRNA can be followed *in vitro* or *in vivo.* The change in secondary structure of the target mRNA can also be checked employing techniques well-known in the art such as **SHAPE** (Poulsen, Line Dahl et al. "SHAPE Selection (SHAPES) enrich for RNA structure signal in SHAPE sequencing-based probing data." RNA (New York, N.Y.) vol. 21,5 (2015): 1042-52. doi:10.1261/rna.047068.114) or **PARIS** (Lu Z, Gong J, Zhang QC. PARIS: Psoralen Analysis of RNA Interactions and Structures with High Throughput and Resolution. Methods Mol Biol. 2018;1649:59-84. doi:10.1007/978-1-4939-7213-5_4).

Optionally, a step of screening for the best performing RNAs of the invention may be carried out, in vitro or in vivo. The skilled person is aware of screening methods, and the present invention includes an example of said screening in the Examples section, where a circular RNA called DM4 was selected as the best performing one. In an embodiment, the screening method is an in vitro screening method and comprises introducing the artificial RNAs of the invention in suitable cells, and identifying whether the disease phenotype is reverted in comparison with a population of control cells that are treated with control RNAs. By "suitable cells" is referred herein as cells that comprise the phenotype of the disease that is to be treated, that is, cells that comprise the pathological target mRNA for which the artificial RNAs of the invention are designed. By "control RNAs" is referred herein to RNAs that have similar features to the RNAs of the invention in terms of length and structure, but that lack the hybridization regions that bind to the pathological target mRNA.

### The artificial circRNA of the invention

In an embodiment, one or more of the artificial RNA(s) of the invention are comprised in a circular molecule, preferably a circular RNA molecule (circRNA), also called herein "the artificial circRNA of the invention".

The artificial circRNA of the invention can be of any length as long as it comprises at least one artificial RNA of the invention that is suitable for disrupting by hybridization one or more secondary structure of one or more target mRNA. Preferably, the circRNA of the invention comprises between 100 and 1000 nucleotides, more preferably between 150 and 800 nucleotides, and even more preferably between 200 and 600 nucleotides.

In a preferred embodiment, the artificial circRNA of the invention comprises 2 or more artificial RNA(s) of the invention, preferably between 6 and 20 artificial RNA(s) of the invention. In a further preferred embodiment, the at least two, and preferably all, of the artificial RNA(s) of the invention comprised in the artificial circRNA of the invention are capable of completely hybridizing with the same target hybridization region. Hence, the artificial circRNA of the invention according to this preferred embodiment would have more probabilities of disrupting the secondary structure of the target mRNA, and would then be more effective in modulating the functionality of the target mRNA.

In another embodiment, the artificial circRNA of the invention, comprises at least two artificial RNAs of the invention, wherein at least two, and preferably all, of their hybridization regions have different nucleotide sequences, i.e., are different from each other in terms of nucleotide sequence. Hence, in this preferred embodiment, the at least two hybridization regions, preferably all of the hybridization regions of the artificial RNAs comprised in the circRNA of the invention, are different from each other, and they all target (are capable of hybridizing) the same target hybridization region ("many-to-one" approach). This way, the artificial circRNA according to this embodiment would have more probabilities of disrupting the secondary structure of a target mRNA, and would then be more effective in modulating the functionality of said target mRNA. Thus, the artificial circRNA of the invention is also capable of inhibiting the pathological function of the target mRNA.

In a further preferred embodiment, the two or more artificial RNAs of the invention comprised in the circular RNA of the invention are:
a) separated by non-hybridization regions of sizes up to 20 nucleotides; or
b) are not separated by non-hybridization regions; or
c) are overlapping.

In a further embodiment, the at least two artificial RNAs of the invention comprised in the circular RNA of the invention are capable of completely hybridizing with at least one target hybridization region comprised in at least one secondary structure of the target mRNA.

The design of the artificial circRNA of the invention can be done once the artificial RNA of the invention have been designed as explained above. The skilled person would merely need to design a circular RNA comprising one or more of the designed artificial RNAs of the invention, separated by random nucleotide sequences. The structure of the designed artificial circRNAs of the invention is very flexible. Generally, it contains at least one, preferably several, artificial RNA(s) whose hybridization region(s) hybridize with at least one, preferably several, target hybridization regions present in at least one secondary structure comprised in the target mRNA. These preferably several hybridization regions in the artificial circRNA of the invention are preferably separated by sequences that are quasi-random, in the sense that they do not perform any functions other than separate the preferably several hybridization regions, and to allow for the observance of the design constraints (low secondary structure, specific GC content, etc.).

However, as explained above, these preferably several hybridization regions of the artificial RNAs comprised in the artificial circRNA of the invention, that target the same target hybridization region within a secondary structure are preferably designed to be different from each other. This is possible since RNA allows for three different base-pairing types: G-C, A-U and G-U.

Since the artificial circRNA of the invention comprises one or more of the artificial RNA of the invention, their mode of action is the same. Thus, the embodiments applicable to the artificial RNAs of the invention and the target mRNA explained above are also applicable to the circular RNA of the invention.

Thus, in an embodiment, the present invention provides an artificial circular RNA molecule comprising one or more, preferably two or more, of the artificial RNAs of the invention, wherein each of the artificial RNAs of the invention are capable of hybridizing to a target mRNA (that can be the same or different), wherein the target mRNA is characterized comprising:
(a) a sequence of nucleotides, preferably 3-5 nucleotides, that is repeated at least 25 times, preferably at least 50 times, and
(b) at least one secondary structure comprising:
   (i) a hairpin loop formed by at least part of the repeated sequence of nucleotides of (a), and
   (ii) at least one target hybridization region which comprises a single-stranded region of at least 2 nucleotides, preferably 3 nucleotides or more, preceded or followed by a double-stranded region of at least 5 nucleotides, preferably 10 nucleotides or more; and
wherein each artificial RNAs of the invention comprised in the circular RNA molecule comprises at least one hybridization region that completely hybridizes with the at least one target hybridization region and disrupts by hybridization the secondary structure of the target mRNA. Similar as explained above, the mRNA targeted by the circular RNA of the invention is a pathological target mRNA, so that it is characterized by causing a disease or an increased risk of having a disease due to the presence of pathological nucleotide repeat expansions.

In an embodiment, the circRNA of the invention comprises one or more of the artificial RNA of the invention that are capable of disrupting by hybridization a secondary structured formed by the CUG trinucleotide expansions present in a pathological target DMPK mRNA. In an embodiment, the circRNA of the invention comprises one or more of the artificial RNAs of the invention designed against a target hybridization region comprising or consisting of SEQ ID NO: 76, 77, 78 or 79.

In an embodiment, the circRNA of the invention comprises one or more of the artificial RNA of the invention that are capable of disrupting by hybridization a secondary structured formed by the CUG trinucleotide expansions present in a pathological target DMPK mRNA, wherein said one or more artificial RNAs are selected from the list of RNAs that comprise or consist of SEQ ID NOs: 9-56, or SEQ ID NOs: 73, 74 and 75, or a sequence that has at least 75%, 80%, 85%, 90%, 91%, 92%, 93%, 94%, 95%, 96%, 97%, 98%, or 100% sequence identity to SEQ ID NO: 9-56 or 73-75, respectively.

In an embodiment, the circRNA of the invention comprises the artificial RNAs of SEQ ID NO: 9, 10, 11, 12, 13, and 14 (circRNA DM1 Target1_GU0_Sol0). In an embodiment, the circRNA of the invention comprises the artificial RNAs of SEQ ID NO: 15, 16, 17, 18, 19, and 20 (circRNA DM4 Target1 GU1_Sol3). In an embodiment, the circRNA of the invention comprises the artificial RNAs of SEQ ID NO: 21, 22, 23, 24, 25, and 26 (circRNA DM5 Target3_GU0_Sol0). In an embodiment, the circRNA of the invention comprises the artificial RNAs of SEQ ID NO: 27, 28, 29, 30, 31, 32 (circRNA DM6 Target3_GU1_Sol0). In an embodiment, the circRNA of the invention comprises the artificial RNAs of SEQ ID NO: 33, 34, 35, 36, 37 and 38 (circRNA DM11 Target2_GU1_Sol0). In an embodiment, the circRNA of the invention comprises the artificial RNAs of SEQ ID NO: 39 ,40, 41, 42, 43 and 44 (circRNA DM15 Target1&3_GU1_Sol0). In an embodiment, the circRNA of the invention comprises the artificial RNAs of SEQ ID NO: 45, 46, 47, 48, 49 and 50 (circRNA DM24 Target3&2_GU1_Sol3). In an embodiment, the circRNA of the invention comprises the artificial RNAs of SEQ ID NO: 51, 52, 53, 54, 55 and 56 (circRNA DM26 Target1&3&2_GU1_Sol0:). Preferably, the artificial RNAs of the invention comprised in the artificial cirRNAs of the invention are separated from each other by random sequences, preferably by random sequences of between 15-25 nucleotides.

In an embodiment, the circRNA of the invention comprises the artificial RNAs of:
- SEQ ID NO: 73 and 74,
- SEQ ID NO: 73 and 75,
- SEQ ID NO: 74 and 75, or
- SEQ ID NO: 73, 74 and 75,
wherein the artificial RNAs of the invention are separated from each other by random sequences, preferably by random sequences of between 15-25 nucleotides.

In an embodiment, the circRNA of the invention comprises the following artificial RNAs of the invention, in a 5' to 3' direction:
- SEQ ID NO: 73 and 74 (circRNA opDM1 T1T3wc_A)
- SEQ ID NO: 73 and 75 (circRNA opDM5 T1T2wc_A)
- SEQ ID NO: 75 and 73 (circRNA opDM7 T2T1wc_A)
- SEQ ID NO: 74 and 75 (circRNA opDM9 T3T2wc_A)
- SEQ ID NO: 75 and 74 (circRNA opDM12 T2T3wc_B)
- SEQ ID NO: 73, 74 and 75 (circRNA opDM13 T1T3T2wc_A)
- SEQ ID NO: 75, 73 and 74 (circRNA opDM21 T2T1T3wc_A), or
- SEQ ID NO: 75, 74 and 73 (circRNA opDM23 T2T3T1wc_A or opDM24 T2T3T1wc_A), wherein the artificial RNAs of the invention are separated from each other by random sequences, preferably by random sequences of between 15-25 nucleotides.

In an embodiment, the circRNA of the invention comprises or consists of SEQ ID NOs: 1-8 or 64-72, or a sequence that has at least 75%, 80%, 85%, 90%, 91%, 92%, 93%, 94%, 95%, 96%, 97%, 98%, or 100% sequence identity to SEQ ID NO: 1-8 or 64-72, respectively.

### The artificial DNAs of the invention

In an embodiment of the first aspect, the present invention also provides artificial DNA molecules that encode (i.e., upon transcription, they give rise to) the artificial RNAs, including the artificial circular RNAs, of the invention. These DNA molecules are also called herein the "artificial DNAs of the invention".

### Compositions of the invention

In a **second aspect,** the present invention provides a composition, also called herein "the composition of the invention", comprising the artificial RNA of the invention, or the artificial circRNA of the invention, or the artificial DNAs of the invention, described above.

Preferably, the composition is a pharmaceutical composition, and preferably comprises the artificial RNA or circRNA or DNA of the present invention, in any of the embodiments, and one or more pharmaceutically acceptable carriers.

Where clinical applications are contemplated, pharmaceutical compositions will be prepared in a form appropriate for the intended application. Generally, this will entail preparing compositions that are essentially free of pyrogens, as well as other impurities that could be harmful to humans or animals.

Colloidal dispersion systems, such as macromolecule complexes, nanocapsules, microspheres, beads, and lipid-based systems including oil-in-water emulsions, micelles, mixed micelles, and liposomes, may be used as delivery vehicles for artificial RNAs, such as circular RNAs. Commercially available fat emulsions that are suitable for delivering the nucleic acids of the disclosure to tissues include Intralipid, Liposyn, Liposyn II, Liposyn III, Nutrilipid, and other similar lipid emulsions.

One will generally desire to employ appropriate salts and buffers to render delivery vehicles stable and allow for uptake by target cells. Buffers also will be employed when recombinant cells (e.g., transfected *ex vivo* with an artificial RNA, such as a circular RNA) are introduced into a patient. Aqueous compositions of the present disclosure comprise an effective amount of the delivery vehicle, dissolved or dispersed in a pharmaceutically acceptable carrier or aqueous medium.

The phrases "pharmaceutically acceptable" or "pharmacologically acceptable" refers to molecular entities and compositions that do not produce adverse, allergic, or other untoward reactions when administered to an animal or a human. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art.

Except insofar as any conventional media or agent is incompatible with the active ingredients of the present disclosure, its use in therapeutic compositions is contemplated. Supplementary active ingredients also can be incorporated into the compositions, provided they do not inactivate the nucleic acids of the compositions.

As for the administration, at least one therapeutically effective cycle of treatment with an artificial RNA or circRNA or DNA of the invention may be administered to a subject for treatment of a disease caused by nucleotide repeat expansions. By "therapeutically effective dose or amount" of a composition comprising an artificial RNA or circRNA or DNA of the invention is intended an amount that, when administered as described herein, brings about a positive therapeutic response, such as improved recovery from the treated condition.

Multiple therapeutically effective doses of compositions comprising artificial RNA or circRNA or DNA of the invention, and/or one or more other therapeutic agents, will be administered. The compositions of the present invention are typically, although not necessarily, administered via injection (subcutaneously, intravenously, intra-arterially, or intramuscularly), by infusion, or locally. Additional modes of administration are also contemplated, such as intraperitoneal, intrathecal, intratumor, intralymphatic, intravascular, intralesion, transdermal, and so forth. In some embodiments, the pharmaceutical composition comprising the artificial RNA or circRNA or DNA of the invention, is administered locally. The pharmaceutical compositions comprising the artificial RNA or circRNA or DNA of the invention, and other agents may be administered using the same or different routes of administration in accordance with any medically acceptable method known in the art.

In a **third aspect,** the present invention provides a kit comprising the artificial RNA or circRNA or DNA of the invention, in any of its embodiments, or comprising the composition of the present invention, and instructions for using the artificial RNA or circRNA or DNA or composition.

Hence, any of the artificial RNAs or circRNA or DNA and/or compositions described herein may be included in a kit. For example circular RNAs as disclosed herein may be included in a kit. The kit may also include one or more transfection reagents to facilitate delivery of the artificial RNAs to cells. Such kits may also include components that preserve the polynucleotides or that protect against their degradation. Such components may be RNAse-free or protect against RNAses.

Such kits generally will comprise, in suitable means, distinct containers for each individual reagent or solution. The kit may comprise one or more containers holding the artificial RNAs and other agents. Suitable containers for the compositions include, for example, bottles, vials, syringes, and test tubes. Containers can be formed from a variety of materials, including glass or plastic. A container may have a sterile access port (for example, the container may be a vial having a stopper pierceable by a hypodermic injection needle).

The kit can further comprise a container comprising a pharmaceutically acceptable buffer, such as phosphate-buffered saline, Ringer's solution, or dextrose solution. It can also contain other materials useful to the end-user, including other pharmaceutically acceptable formulating solutions such as buffers, diluents, filters, needles, and syringes or other delivery devices. The delivery device may be pre-filled with the compositions.

The kit can also comprise a package insert containing written instructions for methods of treating the diseases with the artificial RNAs or DNAs of the present invention. The package insert can be an unapproved draft package insert or can be a package insert approved by the Food and Drug Administration (FDA) or other regulatory body.

In a **fourth aspect,** the present invention provides the artificial RNA or circRNA or DNA of the present invention, in any of its embodiments, or the composition or the kit of the present invention for use as a medicament. Preferably, the present invention provides the artificial RNA or circRNA or DNA of the present invention, in any of its embodiments, or the composition or the kit of the present invention for use in a method of preventing and/or treating a disease caused by nucleotide repeat expansions.

In an embodiment, the disease is selected from the list consisting of Steinert, myotrophic lateral sclerosis (ALS) and related disorders of the ALS/frontotemporal lobar degeneration (FTLD) spectrum, Spinocerebellar ataxia type 8, fragile X-associated tremor/ataxia, and Huntington's disease.

In an embodiment, the disease is Steinert and the artificial RNA comprises or consists of SEQ ID NOs: 9-56, or SEQ ID NOs: 73, 74 and 75, or a sequence that has at least 75%, 80%, 85%, 90%, 91%, 92%, 93%, 94%, 95%, 96%, 97%, 98%, or 100% sequence identity to SEQ ID NOs: 9-56, or SEQ ID NOs: 73, 74 and 75, respectively. In an embodiment, the disease is Steinert and the artificial circular RNA comprises or consists of SEQ ID NOs: 1-8 or 64-72, or a sequence that has at least 75%, 80%, 85%, 90%, 91%, 92%, 93%, 94%, 95%, 96%, 97%, 98%, or 100% sequence identity to SEQ ID NOs: 1-8 or 64-72, respectively.

In **a fifth aspect,** the present invention provides a method of screening for artificial circular RNA of the invention comprising two or more RNAs of the invention and that is capable of disrupting by hybridization at least one secondary structure present in a target mRNA, wherein the secondary structures are defined as comprising:
i. a first region with at least a hairpin loop preceded or followed by a second region of unpaired nucleotides; and
ii. at least one **target hybridization region** which comprises a single-stranded region of at least 2 nucleotides, preferably 3 nucleotides or more preceded or followed by a double-stranded region of at least 5 nucleotides, preferably 10 nucleotides or more, and
wherein the method comprises the steps of:
a) identifying the two or more hybridization regions of the artificial circular RNA as those regions that have a total of between 7 and 100 nucleotides in length, preferably between 10 and 50 nucleotides that, when hybridizing with the at least one target hybridization region, the energy of the hybridization between the two or more hybridization regions and the at least one target hybridization region is more negative than the energy of the secondary structure, thereby disrupting the secondary structure, wherein each of the two or more hybridization regions comprised in the artificial circular RNA, are identified by RNA inverse folding tools, such as NUPACK, RNAifold, or MoiRNAiFold.,
b) designing an artificial circular RNA comprising the two or more hybridization regions identified in a) and capable of disrupting the secondary structure, wherein said artificial circular RNA is between 150 and 800 nucleotides in length, preferably between 200 and 600 nucleotides,
c) optionally selecting the artificial circular RNA as designed in step b) and optionally packaging it into a product.

As indicated above, in step a) of the method according to the fifth aspect or any of its embodiments, the identification of the two or more hybridization regions according to step a) is performed by RNA inverse folding tools such as NUPACK, RNAifold, MoiRNAiFold, wherein the method preferably comprises the steps of:
a) generating one or more nucleotide sequences of the possible two or more hybridization regions that completely hybridize with the at least one target hybridization region comprised in the secondary structure comprised in the target mRNA by providing the RNA inverse folding tools with at least both the secondary structure and the target hybridization region,
b) obtaining as output only the one or more nucleotide sequences of those hybridization regions that, when hybridizing with the target hybridization region, the energy of the hybridization between the two or more hybridization region and the at least one target hybridization region is more negative than the energy of the secondary structure, thereby disrupting the secondary structure.

In a preferred embodiment of the method according to the fifth aspect or any of its embodiments, the method comprises a pre-step that comprises identifying as a secondary structure those structures in a target mRNA comprise:
a) a first region with at least a hairpin loop preceded or followed by a second region of unpaired nucleotides; and
b) at least one target hybridization region which comprises a single-stranded region of at least 2 nucleotides, preferably 3 nucleotides or more preceded or followed by a double-stranded region of at least 5 nucleotides, preferably 10 nucleotides or more.

In a preferred embodiment of the method according to the fifth aspect or any of its embodiments, the method comprises a further step d) of confirming in a biological setting the capacity of the designed artificial circular RNA as designed in step b) of disrupting by hybridization one the secondary structure comprised in the target mRNA.

The present invention is now further illustrated by reference to the following examples which do not intend to limit the scope of the present invention.

### EXAMPLES

**Steinert's disease, or myotonic dystrophy type 1 (DM1),** is a gradually progressive multisystem disorder affecting skeletal muscles, uterine smooth muscle, gastrointestinal smooth muscle, the heart, and the central nervous system. Patients with DM1 have a significantly impaired quality of life and reduced life expectancy. Although it is considered a rare disease (it is estimated to affect 1 in 8000-10,000 people worldwide), recent studies estimate that its prevalence is much higher (4.8/10,000), making it one of the most common rare diseases. At the molecular level, a repeated expansion of CTG of more than 50 units in the 3'UTR region of the gene encoding protein kinase DM1 (DMPK) is the disease-causing mutation. This causes nuclear accumulation of aggregates of secondary structures with repeats in the transcripts of the mutant DMPK gene, resulting in the dysregulation of signaling pathways critical to muscle cell function. The characterization of the molecular pathogenesis of the disease has identified crucial therapeutic targets for the development of new therapies. However, there is currently no specific cure or treatment for DM1, based the therapeutic strategy on palliating the symptoms of the disease, particularly those related to the lungs and heart, which are life-threatening.

### Molecular basis of the Steinert Disease

As mentioned before, Steinert's disease is caused by abnormal expansion of CTG trinucleotide repeats in the 3' region of the DMPK gene, which encodes a serine/threonine kinase. In healthy individuals, the expansion of these trinucleotides ranges from 5 to 37 repeats, while in people with Steinert, it expands between 500 to 3000 repeats. Excessive expansion of CTG causes RNA (ribonucleic acid) resulting from transcription to accumulate in the nucleus and form aberrant hairpin-shaped secondary structures that play a crucial role in the pathogenesis of the disease. These secondary structures in RNA interact with proteins causing their retention in the nucleus and, therefore, the decrease of their activity in processes essential for cellular functioning. One of the key mechanisms in the pathogenesis of Steinert's disease is the interaction of the muscleblind-like protein 1 (MBNL1) with the aberrant secondary structure of RNA. MBNL1 is mostly expressed in skeletal muscle and the nervous system, playing a crucial role in splicing. The retention of MBNL1 causes the appearance of alternative and aberrant splicing events in different genes, which explain several characteristic symptoms of the disease. This process is aggravated due to the retention of RNAs with aberrant structures in the nucleus, which triggers a stress response in the cell that activates different downstream signaling cascades involved in the appearance of defects related to muscle function. An example of these factors is the activation of PKR protein kinase that phosphorylates the CELF1 protein (CUGBP Elav-Like Family Member 1) by activating it. The activation of CELF1 is a process highly regulated by other cell factors such as the GSK3b protein. CELF1 promotes aberrant splicing, alters the expression, stability and translation of RNAs that can lead to proteostasis and / or apoptosis of cells.

Genetic diseases such as Steinert's disease, in which the mutated gene is harmful but the protein is necessary, can only be attacked in 3 ways: 1) by modulating the gene (for example with siRNAs, ASOs, etc.) or by knocking *out* the gene (for example with CRISPr) and adding a wild-type gene at the same time. 2) Doing the above simultaneously, for example with a CRISPr that also adds the homology-corrected part. 3) "Repairing" the harmful effect of the mutation without affecting the gene.

The noctuRNATx strategy disclosed in this invention is within the third type of strategies. The circRNAs of noctuRNATx hybridize with regions of the genome of interest (for example, the region close to the secondary structure formed in RNA in Steinert's disease, or those present in the genome of viruses), so that the secondary target structure of RNA is disrupted and ceases to exert its function. In the case of Steinert's disease, the circRNA designed by noctuRNATx binds to the defective messenger RNA, breaking the secondary structure and preventing the sequestration of the Muscle Blind protein, thus correcting the problems described above (see Figure 2 A). This approach is based on a completely disruptive and novel mechanism of action, which is considered a *first-in-class* therapy. In addition, in the case of a rare disease such as Steinert, the future drug derived from these circRNAs could qualify for orphan drug designation.

To assess the therapeutic potential of our circRNAs to correct the aberrant functions of the DM1-associated pathogenic RNA structures, noctuRNATx first used our in house developed Constraint Programming-based tool to design and generate several circRNAs candidates predicted to hybridize the CUG RNA structures. Within the circRNA designed, we included circRNAs targeting three different regions of the CUG structures (Figure 2 B).
Target hybridization region 1: SEQ ID NO: 76:
   UUGUAGCCGGGAAUGCUGCUGCUGCUGCUG
Target hybridization region 3: SEQ ID NO: 77:
   GCUGCUGCUGCUGGGGGGAUCACAGACCAU
Target hybridization region 2: SEQ ID NO: 78:
   UGCUGCUGCUGCUGCUGCUGCU
Target hybridization region general sequence (SEQ ID NO: 79) comprising SEQ ID NOs 76 (in bold), 77 (in italics) and 78 (underlined): 
UUGUAGCCGGGAAUGCUGCUGCUGCUGCUGCUGCUGCUGCUGCUGCUGC
UGCUGCUGCUGCUGCUGCUGGGGGAUCACAGACCAUUUGUAGCCGGGAAUGCUGC
UGCUGCUGCUGCUGCUGCUGCUGCUGCUGCUGCUGCUGCUGCUGCUGCUGCU
GGGGGAUCACAGACCAU
The target hybridization region general sequence can also be represented as follows: SEQ ID Nos 76 (in bold), 77 (in italics) and 78 (underlined): ......**((((**....**((**.**((**.**((**.**((**.**((**.**((**.**(**(.((.((....)).)).)).)).)).)).*))*.*))*. *))*..*))))*.*((*.........*))*........((((....((.((.((.((.((.((.((.((.((.... )).)).)).)).)).)).)).)).))..)))).((.........))..

Moreover, each circRNA molecule contained six hybridization regions of around 20 to 30 nt long and included small differences within the sequences to increase the probability of binding. To facilitate the screening process, we cloned these sequences into a plasmid that contain at both sides of the cloning site two complementary inverted long terminal repeats (LTR) that allowed the back-splicing of the flanking sequence (Ivanov et al., 2015). In this way, when the corresponding RNA is transcribed in the cell, the LTRs at both side of the cloning RNA will trigger the cellular back-splicing mechanism, generating the circRNAs.

Next, we transfected these different circRNAs into the fibroblast cell line GM03987 derived from a DM1 patient characterized by 500 CTG pathogenic repeats and widely used as a cellular model (Wojtkowiak-Szlachcic et al., 2015). As a control, we used a circRNA targeting a random sequence (NC). Twenty-four hours post-transfection, we harvested the cells and assessed by qPCR their ability to restore the splicing defects of three MBNL-sensitive splicing events, MBNL1 exon 7, MBNL2 exon 7, and Nuclear Receptor Corepressor 2 (NCOR2) exon 45a. The splicing alteration of these three protein exons has been reported to be missregulated in skeletal muscles of DM1 patients and Mbnl1 knockout mice (Nakamori, et al., 2013). All circRNA candidates significantly reduced the percentage of splicing defects in the NCOR2 exon 45a transcript to less than 50% in comparison to cells transfected with the control circRNA while effects on MBLN1 and MBLN2 were more diverse. Altogether, DM4 was the most effective (Figure 3).

Next, we focused on candidate circRNA DM4 to validate the results transfecting directly the circRNA and using myocytes derived from three lines of myoblasts obtained from three DM1 patients and with approximately 300 (isP10), 750 (isP3), 1500 (isP9) CTG repetitions. In this model, closer to the *in vivo* defects, it is possible to test effect on both splicing defects and accumulation of RNA granules in the nucleus visualized by FISH. Notably, DM4 efficiently corrected nuclear foci accumulation in the three different cell lines (Figure 4A) in 5-, 2'5- and 2'8- fold and splicing defects in isP9 cells (Figure 4B).

NoctuRNA also designed and tested a second generation of circRNA candidates against Steinert Disease (Table 4) in fibroblasts. These candidates were optimized taking into account the target region, length and repetitions. We included DM4 as a positive control and an irrelevant circRNA (NC) as a negative control. From this screening, opDM1, opDM7, opDM13, opDM17 y opDM21 were the most effective candidates (Figure 5).

### Materials and methods

### 3.1 circRNA design software

Our circRNA design software is programmed in C++ using the Constraint Programming library OR-TOOLS (https://developers.google.com/optimization/) by Google. The code is available upon demand.

### 3.2 Plasmids

The circoVIR (CV) plasmid was constructed by deleting 1466nt (1910-3375) from pcDNA3-CiRS plasmid (gently provided by Thomas B. Hansen) and inserting unique restriction sites between the splicing and circularization signals. Customized gBlocks (Integrated DNA Technologies) of each DM1-circRNA sequence candidate (Table 1), flanked by BamHI and EcoRI restriction sites, were amplified and then cloned into a BamHI-EcoRI linearized circoVIR plasmid, producing the DM1 pcircRNAs. All constructs were verified by sequencing.

### 3.3 Cell culture

The human fibroblast DM1-patient derived GM03987 cell line, was maintained in Minimum Essential Medium Eagle (EMEM, Sigma-Aldrich) supplemented with 10% non- inactivated fetal bovine serum (FBS) and 1% non-essential amino acids. The myoblasts cell lines isP3, isP9 and isP10 were kindly provided by Dr Gisela Nogales. They were maintained in DMEM supplemented with, Med199 GlutaMAX (Thermofisher), FBS, gentamycin (Thermofisher), fetuin (Life Technology), EGF (Thermofisher), FGF (Thermofisher), insulin (Merck), and dexamethasone (Merck). For differentiation, isP3, isP9 and isP10 cells were grown with Differentiation Media (DMEM, insulin, gentamycin and 2% Horse Serum). All cells were grown in an incubator with 5% CO2 at 37 °C.

### 3.4 Circular RNA plasmids transfection and RNA extraction

2·10⁵ GM03987 cells/well were seeded in 24-well plates the day before transfection. 2 micrograms of each pcircRNA or a control pcircRNA were transfected using Lipofectamine (Invitrogen) following the manufacturer's instructions. A GFP-plasmid control was also transfected to control efficiency of transfection 24 hours later with FACS analysis. Forty-eight hours post transfection, cells were washed with 1X PBS, and lysed with 350 microliters of RLT buffer. RNA extraction was performed using the RNeasy Mini Kit (Qiagen), following manufacturer's instructions.

### 3.5 Retro-transcription and MBNL-sensitive splicing events qPCR

250 nanograms of each extracted RNA sample were reverse transcribed through SuperScript III (Thermofisher) following manufacturer's instruction. 50 nanograms of each cDNA were analyzed by qPCR using Power SYBR^{™} Green PCR Master Mix (Applied biosystems). qPCRs primers against MBNL1 exon 7, MBNL2 exon 7 and NCOR2 exon 45a are specified at table 2.

### 3.7. Circular RNA transfection in myocytes

Prior to seeding, 4 cover-slips were put in each well of a 6 well-plate that needed to be seeded and plates were incubated 30' with UV. The day of the seeding, wells were coated with 1/100 matrigel (Cultek) diluted in DMEM for 30' at 37°C. 10⁵ cells/well were seeded in a 6-well plate from each isP3, isP9 and isP10. The next day, 500 ng of circRNA against DM or 500 ng of an irrelevant circRNA were transfected using Lipofectamine (Invitrogen) following the manufacturer's instructions. The transfection mix was diluted with differentiation media and 2,5 milliliters of the mix was added per well. 5 days post-differentiation, cells were fixed for FISH analyses or treated with Trizol for RNA extraction.

### 3.8 RNA-fluorescence in situ hybridization (FISH)

5 days post-differentiation, cells were washed with pre-warmed PBS and fixed with cold 4% PFA for 15 minutes at room-temperature (RT). After three washings with PBS, and one permeabilization step with 0.3%-triton (diluted in PBS), the coverslips were frozen. After thawing the coverslips and washing three times with 0.1% triton, cells were incubated 10 minutes at RT with acetylation buffer (triethanolamine and acetic anhydride). Then, cells were washed with SSC2x+30% formamide for 10 minutes and incubated for 2h at 37°C with the hybridization buffer containing 1:100 probe (Table 3). Coverslips were washed twice with SSC2x+30% formamide and three-times with PBS. Afterwards, coverslips were incubated 5' RT with DAPI (1/1000) and mounted in a slide using Mowiol. Images were obtained with a SP5 confocal/MP inverted microscope and RNA foci were analyzed using Fiji.

### 3.9 RNA extraction and qPCR

Total RNA was extracted using Trizol (Thermofisher) purification, following manufacturer's instructions. 250 nanograms of each extracted RNA sample were reverse transcribed through SuperScript III (Thermofisher) following manufacturer's instruction. 50 nanograms of each cDNA were analyzed by qPCR using Power SYBR^{™} Green PCR Master Mix (Applied biosystems). qPCRs primers against MBNL1 exon 7 and MBNL2 exon 7 are specified at table 2.

**Table 1. List of DM1 circRNAs sequences**

| **CircRNA of the invention, complete sequence, comprising two or more Artificial RNAs of the invention. The different hybridization regions of the two or more artificial RNAs of the invention are highlighted in bold** | **Hybridization regions of the Artificial RNA of the invention** |
|---|---|
| SEQ ID NO: 1 DM1 Target1_GU0_Sol0: CCCGGUUCCGCACGGUGGUAGUAGCA GCAUUCUUGGCUAUGGGUGGUGGUUC UCUAUACUCGGUAGCAGUAGCAGCAU UUUUGGUUAUAGGCCCAGCUGAGACA AGGGCGGUGGCAGCGGUAGCAUUUCC GGUUAUAAGUUUAAGUUUGCACUGCC CGGCAGUAGUGGUAGCAUUUUCGGUU GUGAACACCAUCGCCCCCCAUGCAGCA GCAGUAGCAGCAUUCUCGGUUACAGA ACUAAGGCACCCACUUAUGGUAGCAGC AGCAGCAUUCCCGGCUAUGAGACAGU C | SEQ ID NO: 9: CGGUGGUAGUAGCAGCAUUCUUGGCU AUGG |
| | SEQ ID NO: 10: CGGUAGCAGUAGCAGCAUUUUUGGUU AUAG |
| | SEQ ID NO: 11: CGGUGGCAGCGGUAGCAUUUCCGGUU AUAA |
| | SEQ ID NO: 12: CGGCAGUAGUGGUAGCAUUUUCGGUU GUGA |
| | SEQ ID NO: 13: CAGCAGCAGUAGCAGCAUUCUCGGUU ACAG |
| | SEQ ID NO: 14: UGGUAGCAGCAGCAGCAUUCCCGGCU AUGA |
| SEQ ID NO: 2 DM4 Target1_GU1_Sol3: CCCGGUUCCGCACAGUAGCAGUAGUG GUGUUCUCGGCUAUAACGUGGUGGUU CUCUAUACCAGCGGUAGCGGUAGCGU UUUUGGCUGCAAUGCCCAGCUGAGAC AAGGCAGCGGUGGCAGCAGCAUUUCU GGUUACAAGGUUUAAGUUUGCACUGC CAGUAGUAGCAGUAGCAUUCCCGGCU GUAACACACCAUCGCCCCCCAUCAGUA GCGGCGGCGGCGUUUCUGGCUACAAG AACUAAGGCACCGACAACAGCAGCAGC GGUGGUGUUUUUGGCUACAAGACAGU C | SEQ ID NO: 15: CAGUAGCAGUAGUGGUGUUCUCGGCU AUAA |
| | SEQ ID NO: 16: CAGCGGUAGCGGUAGCGUUUUUGGCU GCAA |
| | SEQ ID NO: 17: CAGCGGUGGCAGCAGCAUUUCUGGUU ACAA |
| | SEQ ID NO: 18: CAGUAGUAGCAGUAGCAUUCCCGGCU GUAA |
| | SEQ ID NO: 19: CAGUAGCGGCGGCGGCGUUUCUGGCU ACAA |
| | SEQ ID NO: 20: CAGCAGCAGCGGUGGUGUUUUGGCU ACAA |
| SEQ ID NO: 3 DM5 Target3_GU0_Sol0: CCCGGUUCCGCAAUGGUCUGUGAUUU UCUCGGCAGCGGCAGUCGUGGUGGUU CUCUAUACGUGGUCUGUGAUCCUCCC AGUAGUGGCAGUUGCCCAGCUGAGAC AAGGAUGGUCUGUGGUCUUCCCAGCA GUGGCAGUGGUUUAAGUUUGCACUGC AUGGUUUGUGAUCCUCUUAGCAGUAG CGGUCACACCAUCGCCCCCCAUGUGG UCUGUGGUUCUCCCGGCAGCAGUAGC GAACUAAGGCACCCCUCCAUGGUUUGU GGUUUUCCUAGUAGUAGCAGUGACAG UC | SEQ ID NO: 21: AUGGUCUGUGAUUUUCUCGGCAGCGG CAGU |
| | SEQ ID NO: 22: GUGGUCUGUGAUCCUCCCAGUAGUGG CAGU |
| | SEQ ID NO: 23: AUGGUCUGUGGUCUUCCCAGCAGUGG CAGU |
| | SEQ ID NO: 24: AUGGUUUGUGAUCCUCUUAGCAGUAG CGGU |
| | SEQ ID NO: 25: GUGGUCUGUGGUUCUCCCGGCAGCAG UAGC |
| | SEQ ID NO: 26: AUGGUUUGUGGUUUUCCUAGUAGUAG CAGU |
| SEQ ID NO: 4 DM6 Target3_GU1_Sol0: CCCGGUUCCGCAAUGGUCUGUGAUCC CCUUAGUAGUAGUGGCGUGGUGGUUC UCUAUACUAUGGUUUGUGGUUCUCCC GGUGGUAGUGGCGCCCAGCUGAGACA AGGGAUGGUUUGUGAUUUCUCCAGUA GUAGUGGCGUUUAAGUUUGCACUGCC AUGGUUUGUGAUUCCUUUAGUGGCAG CAGCACACCAUCGCCCCCCAUGAUGGU CUGUGAUCUCUCCGGUAGCAGCAGCA ACUAAGGCACCCAUGUCAUGGUCUGU GGUCUUUCUAGCAGUGGUGGCGACAG UC | SEQ ID NO: 27: AUGGUCUGUGAUCCCCUUAGUAGUAG UGGC |
| | SEQ ID NO: 28: AUGGUUUGUGGUUCUCCCGGUGGUAG UGGC |
| | SEQ ID NO: 29: AUGGUUUGUGAUUUCUCCAGUAGUAG UGGC |
| | SEQ ID NO: 30: AUGGUUUGUGAUUCCUUUAGUGGCAG CAGC |
| | SEQ ID NO: 31: AUGGUCUGUGAUCUCUCCGGUAGCAG CAGC |
| | SEQ ID NO: 32: AUGGUCUGUGGUCUUUCUAGCAGUGG UGGC |
| SEQ ID NO: 5 DM11 Target2_GU1_Sol0: CCCGGUUCCGCAAGCGGUAGCAGUAG UAGUAGCAUGGGGAUAAGUAUUGGCC UUUACCGAGCAGUAGUGGCGGCGGUA GUAAAACACUGUCCAUUAAGUCACUGU UAGUAGUAGCGGUGGCAGCAGUAAGA CGCCCGCUUAAACGUCUUUGGUAGCG GCGGUAGCGGCAGCAGCACCUGUCGU CGAUGAGCAGCUGCUAGAGCAGCAGC AGCAGCGGUAGUAAGCCAACAAGAGAA AAACACCCAUAAGCAGUGGUGGUAGUA GCAGCAGACAGUC | SEQ ID NO: 33: |
| | AGCGGUAGCAGUAGUAGUAGCA |
| | SEQ ID NO: 34: |
| | AGCAGUAGUGGCGGCGGUAGUA |
| | SEQ ID NO: 35: |
| | AGUAGUAGCGGUGGCAGCAGUA |
| | SEQ ID NO: 36: |
| | **AGCGGCGGUAGCGGCAGCAGCA** |
| | SEQ ID NO: 37: |
| | **AGCAGCAGCAGCAGCGGUAGUA** |
| | SEQ ID NO: 38: |
| | **AGCAGUGGUGGUAGUAGCAGCA** |
| SEQ ID NO: 6 DM15 Target1&3_GU1_Sol0: CCCGGUUCCGCACAGCGGCAGCAGCA GCAUUUCCGGCUGCAAUGGUGGUUCU CUAUACUGAUGGUUUGUGAUCUCUCU AGUAGCGGUAGCCCCAGCUGAGACAA GGGGCAGUAGCAGUAGUAGUAUUCUC GGUUACAAUUUAAGUUUGCACUGCCAA UGGUCUGUGAUUCUUUCAGUGGCAGU GGCCACCAUCGCCCCCCAUGACAGUA GUAGUAGUGGUAUUCCUGGUUGCAAA CUAAGGCACCGAACACAAUGGUCUGUG AUCCCCUCAGCAGCAGUGGCGACAGU C | SEQ ID NO: 39: CAGCGGCAGCAGCAGCAUUUCCGGCU GCAA |
| | SEQ ID NO: 40: AUGGUUUGUGAUCUCUCUAGUAGCGG UAGC |
| | SEQ ID NO: 41: CAGUAGCAGUAGUAGUAUUCUCGGUU ACAA |
| | SEQ ID NO: 42: AUGGUCUGUGAUUCUUUCAGUGGCAG UGGC |
| | SEQ ID NO: 43: CAGUAGUAGUAGUGGUAUUCCUGGUU GCAA |
| | SEQ ID NO: 44: AUGGUCUGUGAUCCCCUCAGCAGCAG UGGC |
| SEQ ID NO: 7 DM24 Target3&2_GU1_Sol3: CCCGGUUCCGCAAUGGUCUGUGAUUU CUUUGGCAGUAGUGGCCGUGGUGGUU CUCUAUACAGCGGUAGCAGUAGCAGC AGCGUGCCCAGCUGAGACAAGGAUGG UCUGUGAUCCUUUCGGUAGUAGUGGC GGUUUAAGUUUGCACUGCGGCAGUAG UAGUGGCAGUAGCGCACACCAUCGCC CCCCAUAUGGUUUGUGGUUCCUCUAG UAGUGGCAGCGAACUAAGGCACCUAUA GAGUGGCAGCAGUGGUAGCAGUAGAC AGUC | SEQ ID NO: 45: AUGGUCUGUGAUUUCUUUGGCAGUAG UGGC |
| | SEQ ID NO: 46: |
| | **AGCGGUAGCAGUAGCAGCAGCG** |
| | SEQ ID NO: 47: AUGGUCUGUGAUCCUUUCGGUAGUAG UGGC |
| | SEQ ID NO: 48: |
| | **GGCAGUAGUAGUGGCAGUAGCG** |
| | SEQ ID NO: 49: AUGGUUUGUGGUUCCUCUAGUAGUGG CAGC |
| | SEQ ID NO: 50: |
| | **AGUGGCAGCAGUGGUAGCAGUA** |
| SEQ ID NO: 8 DM26 Target1&2&3_GU1_Sol0: CCCGGUUCCGCACAGUGGUAGUAGUA GCAUUUCUGGUUGUGACGUGGUGGUU CUCUAUACAUGGUUUGUGGUUCCCUU AGUGGCGGUAGCUGCCCAGCUGAGAC AAGGAGUGGUGGCGGCGGCAGUA GGUUUAAGUUUGCACUGCCAGUAGCG GUGGUAGUAUUUUUGGUUGUAACACA CCAUCGCCCCCCAUAUGGUUUGUGAU UUCUCUGGUGGCAGUGGCGAACUAAG GCACAACUCCGGUAGUAGUGGUAGUG GUAGUAGACAGUC | SEQ ID NO: 51: CAGUGGUAGUAGUAGCAUUUCUGGUU GUGA |
| | SEQ ID NO: 52: AUGGUUUGUGGUUCCCUUAGUGGCGG UAGC |
| | SEQ ID NO: 53: |
| | **AGUGGUGGCGGCGGCGGCAGUA** |
| | SEQ ID NO: 54: |
| | CAGUAGCGGUGGUAGUAUUUUUGGUU GUAA |
| | SEQ ID NO: 55: AUGGUUUGUGAUUUCUCUGGUGGCAG UGGC |
| | SEQ ID NO: 56: |
| | **GGUAGUAGUGGUAGUGGUAGUA** |

**Table 2. List of qPCR primers**

| Transcript name | Sequence 5'-3' |
|---|---|
| MBNL1 | f:GCTGCCCAATACCAGGTCAAC (SEQ ID NO: 57) |
| | r:TGGTGGGAGAAATGCTGTATGC (SEQ ID NO: 58) |
| MBNL2 | f:TCCTTTACCAAAGAGACAAGCAC (SEQ ID NO: 59) |
| | r:CTCAATGCAGATTCTTGGCATTCC (SEQ ID NO: 60) |
| NCOR2 | f:ACACCCACAACCGGAATGAGCCTG (SEQ ID NO: 61) |
| | r:GGACTTGGCTTTTCGGCTGCTG (SEQ ID NO: 62) |

**Table 3. Probe for RNA FISH**

| Probe | Sequence 5'-3' |
|---|---|
| DM-probe | /5Cy3/CAGCAGCAGCAGCAGCAGCAGCAG CAGCAG (SEQ ID NO: 63) |

**Table 4. List of DM1 optimized circRNAs sequences**

| **CircRNA of the invention, complete sequence, comprising two or more Artificial RNAs of the invention. The different hybridization regions of the two or more artificial RNAs of the invention are highlighted in bold** | **Hybridization regions of the Artificial RNA of the invention** |
|---|---|
| SEQ ID NO: 64: opDM1 T1T3wc_A CCCGGUUCCGCACAUAUGAAGAUAGA CCAGCAGCAGCAGCAGCAUUCCCGGC UACAACUCUAUCCCGUCUCGCUCGAA | SEQ ID NO: 73: CAGCAGCAGCAGCAUUCCCGGCUACA A |
| | SEQ ID NO: 74: AUGGUCUGUGAUCCCCCCAGCAGCAG CAGC |
| UGGUCUGUGAUCCCCCCAGCAGCAGC AGCAUUUUCUCGAUUCGUGACAGUC | |
| SEQ ID NO: 65: opDM5 T1T2wc_A: CCCGGUUCCGCAUAGUUAAAAAAUCCA CAGCAGCAGCAGCAUUCCCGGCUACA ACAGAGUACAACUUGAUCGACCAGCA GCAGCAGCAGCAGCAAGAAAAAAUAG UGGAGACAGUC | SEQ ID NO: 73: CAGCAGCAGCAGCAUUCCCGGCUACA A |
| | SEQ ID NO: 75: |
| | **AGCAGCAGCAGCAGCAGCA** |
| SEQ ID NO: 66: opDM7 T2T1wc_A: CCCGGUUCCGCAAGUUGAUUGAUAUC ACAGCAGCAGCAGCAGCAGCAUAAAC UAGCGUGACGAUGCUCAGCAGCAGCA GCAUUCCCGGCUACAACUCCUCGCUC UUCUGGACAGUC | SEQ ID NO: 75: |
| | **AGCAGCAGCAGCAGCAGCA** |
| | SEQ ID NO: 73: CAGCAGCAGCAGCAUUCCCGGCUACA A |
| SEQ ID NO: 67: opDM9 T3T2wc_A: CCCGGUUCCGCAGCGAGUGUCGUUGA GAUGGUCUGUGAUCCCCCCAGCAGCA GCAGCCCCAUGUAUGGACCCACUAAC AGCAGCAGCAGCAGCAGCAAUGGAAC AGAGCUGUGACAGUC | SEQ ID NO: 74: AUGGUCUGUGAUCCCCCCAGCAGCAG CAGC |
| | SEQ ID NO: 75: |
| | **AGCAGCAGCAGCAGCAGCA** |
| SEQ ID NO: 68: opDM12 T2T3wc_B: CCCGGUUCCGCAUCUCCAACGAUGGU ACAGCAGCAGCAGCAGCAGCAUCACC UAUGUGCCGACUUAAAUGGUCUGUGA UCCCCCCAGCAGCAGCAGCUUUCGUG AGGACUUCGACAGUC | SEQ ID NO: 75: |
| | **AGCAGCAGCAGCAGCAGCA** |
| | SEQ ID NO: 74: AUGGUCUGUGAUCCCCCCAGCAGCAG CAGC |
| SEQ ID NO: 69: opDM13 T1T3T2wc_A: CCCGGUUCCGCAUACUGAAGGAUUUU ACAGCAGCAGCAGCAUUCCCGGCUAC AACCUGACAAUCACCAACGCAAAUGG UCUGUGAUCCCCCCAGCAGCAGCAGC UUUGAUAUAGCGGACAAAAACAGCAG CAGCAGCAGCAGCAAAAAAAUCAUAA GGCGACAGUC | SEQ ID NO: 73: CAGCAGCAGCAGCAUUCCCGGCUACA A |
| | SEQ ID NO: 74: AUGGUCUGUGAUCCCCCCAGCAGCAG CAGC |
| | SEQ ID NO: 75: |
| | **AGCAGCAGCAGCAGCAGCA** |
| SEQ ID NO: 70: opDM21 T2T1T3wc_A: CCCGGUUCCGCAUCUAUCCAGACUUU CCAGCAGCAGCAGCAGCAGCACCGCA GGUCCUCGCACAACUCAGCAGCAGCA GCAUUCCCGGCUACAACUUUUAUACC UAUCAUAGCUAUGGUCUGUGAUCCCC CCAGCAGCAGCAGCUUUUCCUGAUCC ACUGACAGUC | SEQ ID NO: 75: |
| | **AGCAGCAGCAGCAGCAGCA** |
| | SEQ ID NO: 73: CAGCAGCAGCAGCAUUCCCGGCUACA A |
| | SEQ ID NO: 74: AUGGUCUGUGAUCCCCCCAGCAGCAG CAGC |
| SEQ ID NO: 71: opDM23 T2T3T1wc_A: CCCGGUUCCGCAUCAAUGCAUCCUAU CCAGCAGCAGCAGCAGCAGCACCGCA UGCUAAUAUCCCUCCAUGGUCUGUGA UCCCCCCAGCAGCAGCAGCCUUAUUG UCGCAUAGUCUCCCAGCAGCAGCAGC AUUCCCGGCUACAAUUUUCCGACUCA UACGACAGUC | SEQ ID NO: 75: |
| | **AGCAGCAGCAGCAGCAGCA** |
| | SEQ ID NO: 74: AUGGUCUGUGAUCCCCCCAGCAGCAG CAGC |
| | SEQ ID NO: 73: CAGCAGCAGCAGCAUUCCCGGCUACA A |
| SEQ ID NO: 72: opDM24 T2T3T1wc_A: CCCGGUUCCGCAAUAUUCAAUAACGAA CAGCAGCAGCAGCAGCAGCACAAGAU CCUCACCUAAUGCAAUGGUCUGUGAU CCCCCCAGCAGCAGCAGCUGAGUCCC GAGCAAUAGGCGCAGCAGCAGCAGCA UUCCCGGCUACAAUGAAUAUGCAGGU GUGACAGUC | SEQ ID NO: 75: |
| | **AGCAGCAGCAGCAGCAGCA** |
| | SEQ ID NO: 74: AUGGUCUGUGAUCCCCCCAGCAGCAG CAGC |
| | SEQ ID NO: 73: CAGCAGCAGCAGCAUUCCCGGCUACA A |

## Claims

1. An **artificial RNA molecule** capable of hybridizing to a target mRNA, wherein the target mRNA is **characterized by** causing a disease or an increased risk of having a disease and by comprising:
(a) a sequence of nucleotides, preferably 3-5 nucleotides, that is repeated at least 25 times, preferably at least 50 times, and
(b) at least one secondary structure comprising:
(i) a hairpin loop formed by at least part of the repeated sequence of nucleotides of (a), and
(ii) at least one **target hybridization region** which comprises a single-stranded region of at least 2 nucleotides, preferably 3 nucleotides or more, preceded or followed by a double-stranded region of at least 5 nucleotides, preferably 10 nucleotides or more; and
wherein the artificial RNA molecule comprises at least one **hybridization region** that completely hybridizes with the at least one target hybridization region and disrupts by hybridization the secondary structure of the target mRNA.

2. The artificial RNA molecule according to claim 1, wherein the at least one hybridization region comprised in the artificial RNA molecule is **characterized in that** the energy of hybridization between the at least one hybridization region and the at least one target hybridization region is more negative than the energy of the secondary structure comprised in the target mRNA, preferably wherein the energy of hybridization is measured by RNAcofold.

3. The artificial RNA molecule according to any one of claims 1 or 2, wherein the target mRNA is a myotonic dystrophy protein kinase (DMPK) mRNA **characterized by** comprising, in the 3' UTR, (CUG)a trinucleotide repeats, wherein a is 38 or more, preferably 50 or more, and wherein the disease that is caused or that has increased risk of being caused is Steinert disease.

4. The artificial RNA molecule according to claim 3, wherein the at least one target hybridization region comprised in the secondary structure comprises or consists of SEQ ID NO: 76, 77, 78 or 79.

5. The artificial RNA molecule according to claim 4, wherein:
- the at least one target hybridization region comprised in the secondary structure comprises SEQ ID NO: 76, and the hybridization region comprised in the artificial RNA molecule comprises SEQ ID NOs: 9, 10, 11, 12, 13, 14, 15, 16, 17, 18, 19, 20, 39, 41, 43, 51, 54, and 73,
- the at least one target hybridization region comprised in the secondary structure comprises SEQ ID NO: 77, and the hybridization region comprised in the artificial RNA molecule comprises SEQ ID NOs: 21, 22, 23, 24, 25, 26, 27, 28, 29, 30, 31, 32, 40, 42, 44, 45, 47, 49, 52, 55, and 74, or
- the at least one target hybridization region comprised in the secondary structure comprises SEQ ID NO: 78, and the hybridization region comprised in the artificial RNA molecule comprises SEQ ID NOs: 33, 34, 35, 36, 37, 38, 46, 48, 50, 53, 56, and 75.

6. An **artificial circular RNA molecule** comprising one or more, preferably at least two, of the artificial RNA molecule as defined in any one of claims 1 to 5.

7. The artificial circular RNA molecule of claim 6, wherein the artificial circular RNA molecule comprises between 6 and 20 artificial RNA molecules as defined in any one of claims 1 to 5.

8. The artificial circular RNA molecule of any one of claims 6 or 7, wherein the artificial circular RNA molecule comprises between 150 and 800 nucleotides, preferably between 200 and 600 nucleotides.

9. The artificial circular RNA molecule of any one of claims 6 to 8, comprising or consisting of any one of SEQ ID Nos: 1-8 or 64-72.

10. An **artificial DNA molecule** that, upon transcription, encodes the artificial RNAs defined in any one of claims 1 to 5, or the artificial circular RNA molecules as defined in any one of claims 6 to 9.

11. A **composition** comprising the artificial RNA or circular RNA or DNA molecules as defined in any of the previous claims.

12. A kit comprising the artificial RNA or circular RNA or DNA molecule as defined in any of claims 1 to 10, or comprising the composition as defined in claim 11, and instructions for using the artificial RNA or circular RNA or DNA or composition.

13. The artificial RNA or circular RNA or DNA molecule as defined in any of claims 1 to 10, the composition as defined in claim 11, or the kit as defined in claim 12, for use as a medicament.

14. An artificial RNA molecule as defined in any of claims 3 to 5, or an artificial circular RNA or artificial DNA encoding the same, or a composition comprising them, for use in a method of preventing and/or treating Steinert's disease.

15. An artificial RNA molecule comprising one or more of SEQ ID NO: 9-56, or SEQ ID NOs: 73, 74 or 75, for use in a method of preventing and/or treating Steinert's disease.
